# EUROPEAN PATENT SPECIFICATION

(11) **EP 2 868 281 B1**
(45) Date of publication and mention of the grant of the patent: **03.04.2019**
(21) Application number: 14189142.4
(22) Date of filing: 16.10.2014
(51) Int. Cl.: A61B 17/072, A61B 17/00

(54) **Surgical Instrument, Loading Unit and Fasteners for Use Therewith**
Chirurgisches Instrument, Ladeeinheit und Halterungen zur Verwendung damit
Instrument chirurgical, unité de chargement et attaches à utiliser avec celui-ci

(30) Priority: 17.10.2013 US 201314056198
(43) Date of publication of application: 06.05.2015
(73) Proprietor: Covidien LP, Mansfield, MA 02048 (US)
(72) Inventor: Estrella, Russell, Naugatuck, CT Connecticut 06770 (US)
(74) Representative: Soames, Candida Jane

(56) References cited:
- EP-A2- 2 540 231
- WO-A2-00/64360
- US-A1- 2008 065 154
- US-A1- 2010 213 238

## Description

### BACKGROUND

### Technical field

The present disclosure relates generally to instruments for surgically joining tissue and, more specifically, to surgical instruments, loading units and fasteners for use therewith.

### Background of Related Art

Various types of surgical instruments used to surgically join tissue are known in the art, and are commonly used, for example, for closure of tissue or organs in transection, resection, anastomoses, for occlusion of organs in thoracic and abdominal procedures, and for electrosurgically fusing or sealing tissue.

One example of such a surgical instrument is a surgical stapling instrument, which may include an anvil assembly, a cartridge assembly for supporting an array of surgical staples, an approximation mechanism for approximating the cartridge and anvil assemblies, and a firing mechanism for ejecting the surgical staples from the cartridge assembly.

US 2010/213238 A1 discloses a surgical instrument having a curved cartridge and anvil assembly, including staples disposed in staple retention slots and a curved slot through which a cutting edge may travel. The cartridge may comprise staples of varying leg lengths, e.g. at different positions across the width of the cartridge.

Using a surgical stapling instrument, it is common for a surgeon to approximate the anvil and cartridge members. Next, the surgeon can fire the instrument to emplace staples in tissue. Additionally, the surgeon may use the same instrument or a separate instrument to cut the tissue adjacent or between the row(s) of staples.

### SUMMARY

The present invention provides a loading unit for use with a surgical instrument, the loading unit comprising: a proximal body portion configured to engage a portion of a surgical instrument and defining a longitudinal axis; a pair of jaw members disposed adjacent the proximal body portion and extending generally distally therefrom, at least one of the jaw members being movable with respect to the other between an open position and an approximated position for engaging body tissue therebetween, the pair of jaw members including a first jaw member and a second jaw member; and a plurality of staples disposed at least partially within the second jaw member, wherein at least some of the staples disposed in a first portion of the second jaw member having a smaller height than at least some of the staples disposed in a second portion of the second jaw member; wherein the second jaw member further comprises a slot configured to allow a knife to travel at least partially therealong, wherein the slot is curved with respect to the longitudinal axis; characterised in that the first portion of the second jaw member is disposed (i) on an inner side of the curvature of the slot and (ii) farther proximally than the second portion of the second jaw member, and the second portion of the second jaw member is further disposed on an outer side of the curvature of the slot.

The present disclosure relates to a surgical instrument for surgically joining tissue. The instrument includes a handle assembly, an endoscopic portion, a pair of jaw members, and a plurality of staples. The endoscopic portion extends distally from the handle assembly and defines a longitudinal axis. The pair of jaw members is disposed adjacent a distal end of the endoscopic portion and extends generally distally therefrom. Each of the jaw members is longitudinally curved with respect to the longitudinal axis. At least one of the jaw members is movable with respect to the other between an open position and an approximated position for engaging body tissue therebetween. The pair of jaw members includes a first jaw member and a second jaw member. The plurality of staples is disposed at least partially within the second jaw member. Each of the staples includes a pair of legs depending from a backspan. Each leg includes a staple tip defining a first angle α1 between about 25° and about 35°.

In disclosed examples, the first angle α1 is approximately equal to 30°.

In disclosed examples, each staple tip includes a single staple point. Here, it is disclosed that each staple point is aligned with an inner edge of the respective staple leg. It is further disclosed that each staple point is aligned with an outer edge of the respective staple leg.

In disclosed examples, each staple tip defines a second angle α2 between about 25° and about 35°.

In disclosed examples, at least some of the staples disposed within a proximal portion of the second jaw member include a smaller height than at least some of the staples disposed distally of the staples disposed within the proximal portion of the second jaw member.

The present disclosure also relates to a surgical instrument for surgically joining tissue comprising a handle assembly, an endoscopic portion, a pair of jaw members, and a plurality of staples. The endoscopic portion extends distally from the handle assembly and defines a longitudinal axis. The pair of jaw members is disposed adjacent a distal end of the endoscopic portion and extends generally distally therefrom. Each of the jaw members is longitudinally curved with respect to the longitudinal axis. At least one of the jaw members is movable with respect to the other between an open position and an approximated position for engaging body tissue therebetween. The pair of jaw members includes a first jaw member and a second jaw member. The plurality of staples is disposed at least partially within the second jaw member. Each of the staples includes a pair of legs depending from a backspan, and each leg includes a staple tip defining a first angle α1 and a second angle α2.

In disclosed examples, each staple tip includes a single staple point. Here, it is disclosed that each staple point is disposed between an extension of an inner edge and an outer edge of the respective staple leg.

In disclosed examples, the first angle α1 is between about 20° and 50°. Here, it is disclosed that the second angle α2 is between about 20° and 50°.

In disclosed examples, the first angle α1 is between about 25° and 35°.

In disclosed examples, the first angle α1 and the second angle α2 are approximately equal to each other.

In disclosed examples, the first angle α1 and the second angle α2 are different from each other.

In disclosed examples, at least some of the staples disposed in a proximal portion of the second jaw member include a smaller height than at least some of the staples disposed distally of the staples disposed in the proximal portion of the second jaw member.

The present disclosure also relates to a loading unit for use with a surgical instrument. The loading unit comprise a proximal body portion, a pair of jaw members, and a plurality of staples. The proximal body portion is configured to engage a portion of a surgical instrument and defines a longitudinal axis. The pair of jaw members is disposed adjacent the proximal body portion and extends generally distally therefrom. At least one of the jaw members is movable with respect to the other between an open position and an approximated position for engaging body tissue therebetween. The pair of jaw members includes a first jaw member and a second jaw member. The plurality of staples is disposed at least partially within the second jaw member. At least some of the staples disposed in a first portion of the second jaw member have a smaller height than at least some of the staples disposed in a second portion of the second jaw member.

In disclosed examples, the first portion of the second jaw member is disposed farther proximally than the second portion of the second jaw member.

In disclosed examples, the second jaw member further comprises a slot configured to allow a knife to travel at least partially therealong. Here, it is disclosed that the first portion of the second jaw member is disposed on a first lateral side of the slot, and the second portion of the second jaw member is disposed on a second lateral side of the slot. It is further disclosed that the slot is curved with respect to the longitudinal axis. In examples, the first portion of the second jaw member is disposed on an inner side of the curvature of the slot, and the second portion of the second jaw member is disposed on an outer side of the curvature of the slot.

In disclosed examples, each lateral side of the slot of the second jaw member includes an outer row of staple retention slots, an inner row of staple retention slots and a middle row of staple retention slots, the inner row of staple retention slots is closest to the slot. Here, the first portion of the second jaw member includes the outer rows of staple retention slots and the middle rows of staple retention slots, and the second portion of the second jaw member includes the inner rows of staple retention slots.

In disclosed examples, each lateral side of the slot of the second jaw member includes an outer row of staple retention slots, an inner row of staple retention slots and a middle row of staple retention slots, the inner row of staple retention slots is closest to the slot. Here, the first portion of the second jaw member includes the outer row of staple retention slots, the middle row of staple retention slots and the inner row of staple retention slots on an inside portion of the curvature of the slot. The first portion of the second jaw member also includes the inner row of staple retention slots on an outside portion of the curvature of the slot. The second portion of the second jaw member includes the middle row of staple retention slots and the outer row of staple retention slots on the outside portion of the curvature of the slot. Here, it is disclosed that the staples disposed in the outer row of staple retention slots on the outside portion of the curvature of the slot are larger than the staples disposed in the middle row of staple retention slots on the outside portion of the curvature of the slot.

In disclosed examples, at least some of the staples include a pair of legs depending from a backspan, and each leg includes a staple tip defining a first angle α1, and wherein the first angle α1 is between about 25° and about 35°.

In disclosed examples, at least some of the staples include a pair of legs depending from a backspan, and each leg includes a staple tip defining a first angle α1 and a second angle α2.

### BRIEF DESCRIPTION OF FIGURES

Various examples, of the presently disclosed surgical instrument are disclosed herein with reference to the drawings, wherein:
Figure 1 is a perspective view of a surgical stapling instrument including a loading unit in accordance with the present disclosure;
Figure 1A is a perspective view of another type of surgical stapling instrument including the loading unit of Figure 1 in accordance with an example of the present disclosure;
Figure 2 is a perspective view of a handle assembly of the surgical stapling instrument of Figure 1A;
Figure 3 is a perspective view of the loading unit of Figures 1 and 1A;
Figure 4 is an enlarged view of the area of detail of Figures 1 and 1A;
Figure 5 is a top view of the loading unit of Figures 3 and 4;
Figure 6 is a side view of the loading unit of Figures 3-5, illustrated with a cartridge assembly in the open position;
Figure 7 is a perspective, partial cross-sectional view of the loading unit of Figures 3-6;
Figure 8 is a transverse cross-sectional view of the loading unit of Figures 3-7;
Figure 9 is a longitudinal cross-sectional view of a portion of the loading unit of Figures 3-8;
Figure 10 is a perspective assembly view of the loading unit of Figures 3-9;
Figure 11 is a perspective view of a drive assembly and dynamic clamping member of the loading unit of Figures 3-10;
Figure 12 is an enlarged view of the area of detail of Figure 11;
Figure 13 is a perspective assembly view of the drive assembly and dynamic clamping member of Figures 11 and 12;
Figures 14-17 are various views of the dynamic clamping member according to an example of the present disclosure;
Figure 17A is a rear view of another example of a dynamic clamping member according to another example of the present disclosure;
Figure 17B is a perspective view of another example of a dynamic clamping member according to another example of the present disclosure;
Figures 18-20 are various views of an actuation sled in accordance with an example of the present disclosure;
Figures 21 and 22 are perspective views of staples and staple pushers in accordance with examples of the present disclosure;
Figures 23-25 are perspective views of various staple pushers in accordance with examples of the present disclosure;
Figure 26 is a perspective view of a tissue stop for use with the loading unit of Figures 3-10;
Figure 27 is a cross-sectional view of the tissue stop of Figure 26 coupled to the loading unit;
Figures 28-30 are perspective views of the loading unit of Figures 3-10 interacting with a layer of tissue at various stages of operation of the loading unit;
Figure 31 is a transverse cross-sectional view of the surgical instrument taken across a portion of the actuation sled in accordance with an example of the present disclosure;
Figure 32 is a transverse cross-sectional view of the surgical instrument of Figure 30 taken across a portion of the drive assembly;
Figure 33 illustrates a staple for use with the surgical instrument and loading unit in accordance with an example of the present disclosure;
Figure 34 illustrates a portion of the staple as indicated in Figure 33;
Figure 35 illustrates a staple for use with the surgical instrument and loading unit in accordance with an example of the present disclosure;
Figure 36 illustrates a portion of the staple as indicated in Figure 35;
Figure 37 illustrates a staple for use with the surgical instrument and loading unit in accordance with an example of the present disclosure;
Figure 38 illustrates a portion of the staple as indicated in Figure 37; and
Figure 39 illustrates a top view of a cartridge for use with the surgical instrument and loading unit in accordance with embodiments of the present invention.

### DETAILED DESCRIPTION

Examples of the presently disclosed surgical instrument, and loading unit for use therewith, are described in detail with reference to the drawings, wherein like reference numerals designate corresponding elements in each of the several views. As is common in the art, the term 'proximal" refers to that part or component closer to the user or operator, e.g., surgeon or physician, while the term "distal" refers to that part or component farther away from the user.

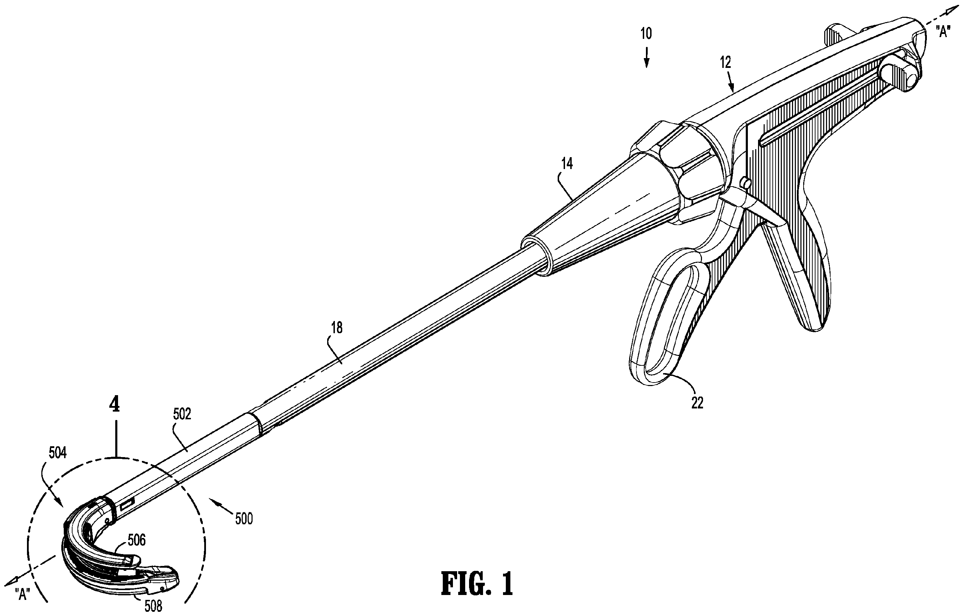

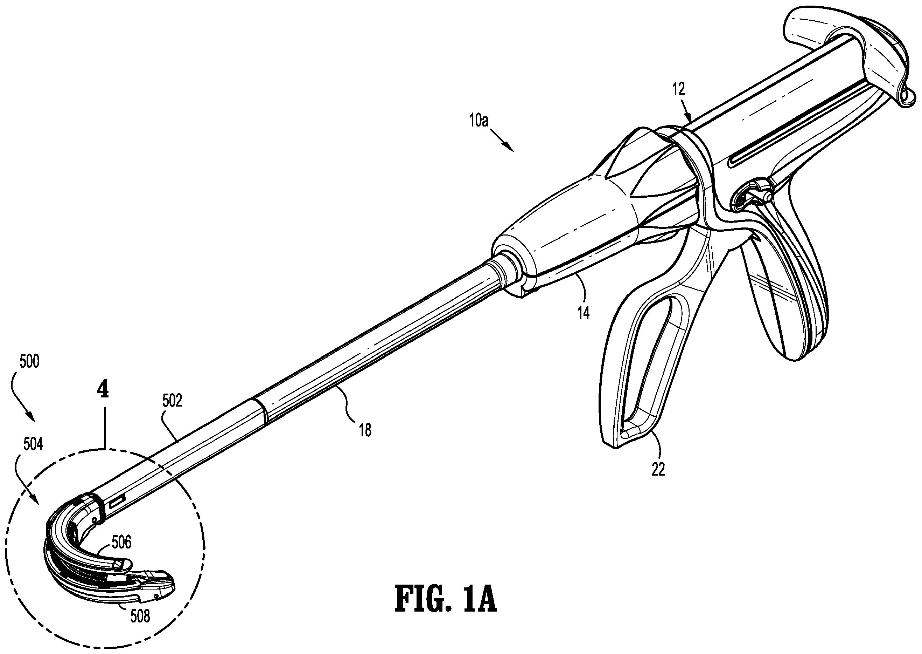

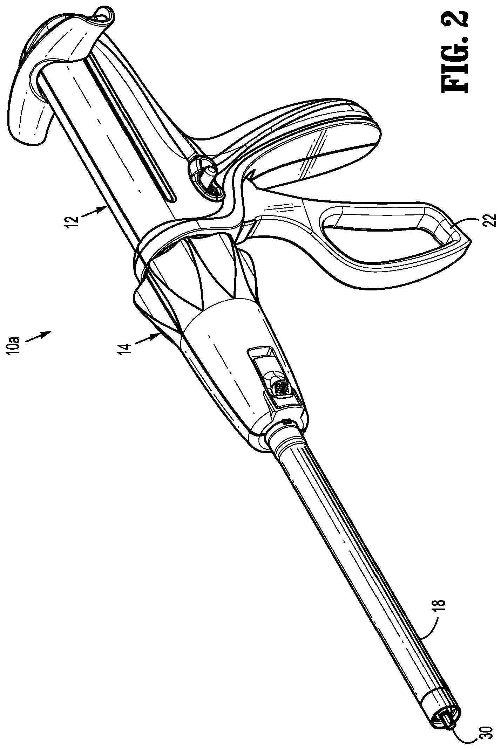

A first type of surgical stapling instrument of the present disclosure is indicated as reference numeral 10 in Figure 1. Another type of surgical stapling instrument of the present disclosure is indicated as reference numeral 10a in Figures 1A and 2. Additionally, while not explicitly shown, the present application also relates to surgical stapling instruments having parallel jaw members and to electrosurgical instruments used to join tissue. Collectively, all surgical instruments (including surgical stapling instruments 10 and 10a) are referred to herein as "surgical instrument" and referred to as reference numeral 10. Similarly, several features that are common to both surgical stapling instruments are collectively referred to as the same reference number (e.g., handle assembly 12, rotation knob 14, and endoscopic portion 18). Further details of an endoscopic surgical stapling instrument are described in detail in commonly-owned U.S. Patent No. 6,953,139 to Milliman et al..

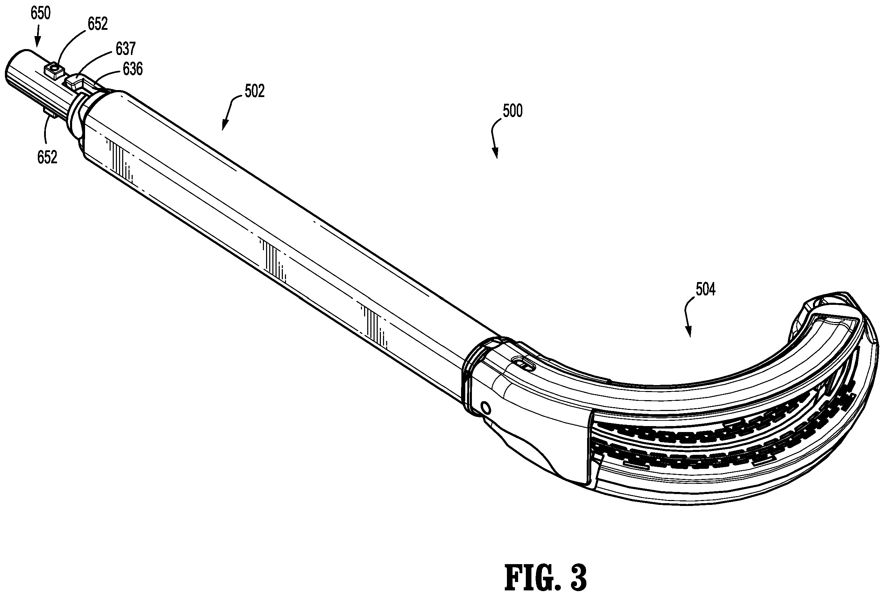

A loading unit 500 (e.g., a disposable loading unit or a reusable loading unit) for use with surgical instrument 10 is shown in FIGS. 3-10 and 28-30. Loading unit 500 is attachable to an elongated or endoscopic portion 18 of surgical instrument 10, e.g., to allow surgical instrument 10 to have greater versatility. Loading unit 500 may be configured for a single use, and/or may be configured to be used more than once. Examples of loading units for use with a surgical stapling instrument are disclosed in commonly-owned United States Patent No. 5,752,644 to Bolanos et al.. The loading unit shown includes a proximal body portion that is attachable to an elongated portion of a surgical instrument having a handle assembly. However, the tool assembly can be incorporated in a surgical instrument in which a staple cartridge is removable and replaceable and does not include a detachable portion of the elongated portion of the instrument.

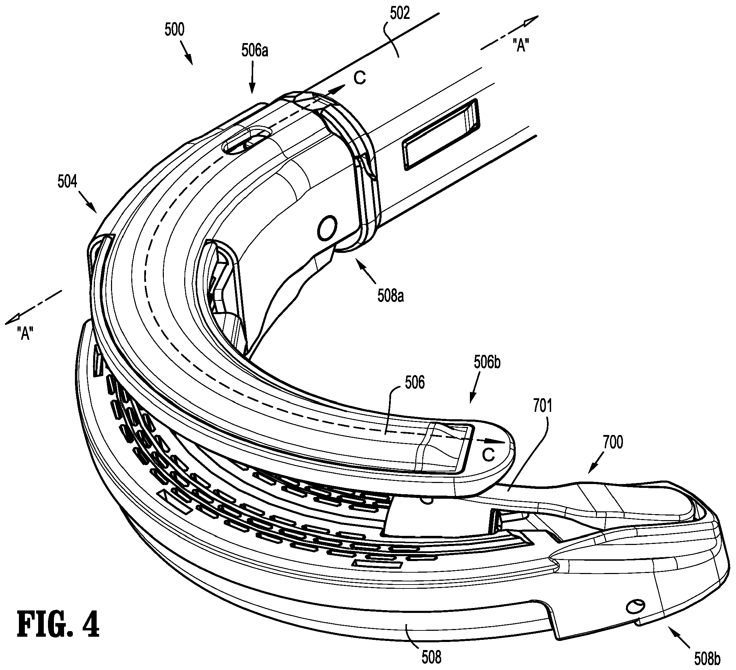

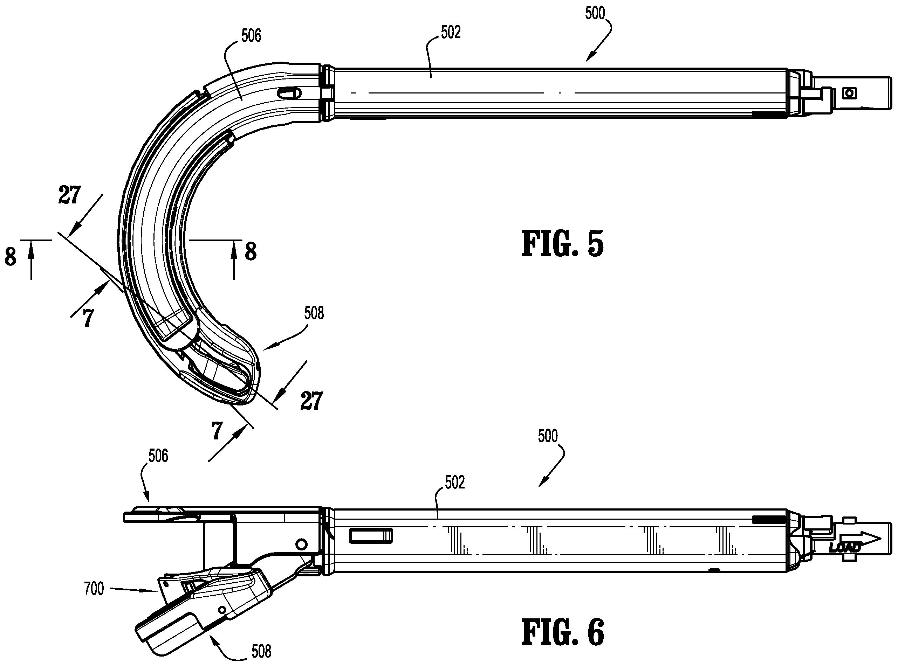

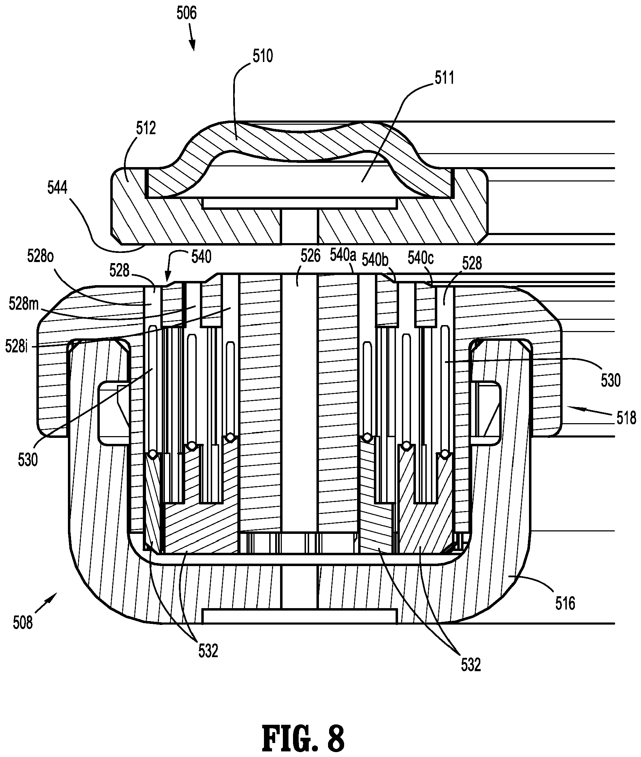

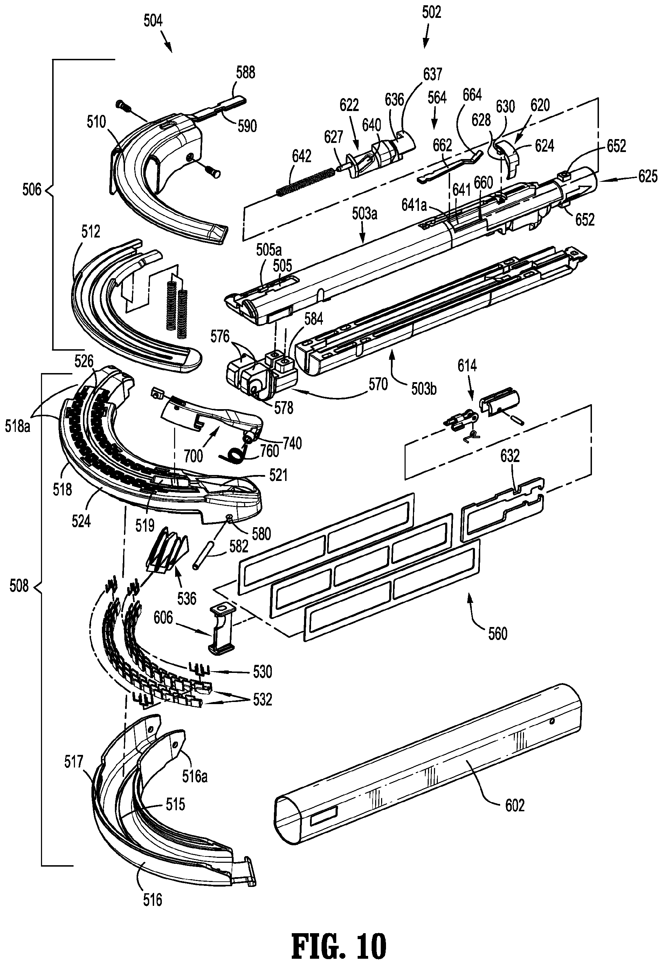

Loading unit 500 includes a proximal body portion 502 and a tool assembly 504. Proximal body portion 502 defines a longitudinal axis "A-A," and is releasably attachable to a distal end of elongated body portion 18 of surgical instrument 10. Tool assembly 504 includes a pair of jaw members including an anvil assembly 506 and a cartridge assembly 508. One jaw member is pivotal in relation to the other. In the illustrated examples, cartridge assembly 508 is pivotal in relation to anvil assembly 506 and is movable between an open or unclamped position (e.g., FIGS. 4 and 6) and a closed or approximated position (e.g., FIG. 8). Cartridge assembly 508 is urged in the open position via a biasing member, e.g., a pair of compression springs 533 disposed between anvil cover 510 and cartridge 518 (see FIG. 10).

With reference to FIGS. 1 and 10, for example, tool assembly 504 includes anvil assembly 506 and cartridge assembly 508. As shown, each of anvil assembly 506 and cartridge assembly 508 is longitudinally curved. That is, anvil assembly 506 and cartridge assembly 508 are curved with respect to the longitudinal axis "A-A" defined by proximal body portion 502. As used herein with respect to curved parts of the surgical instrument 10 of the present disclosure, the term "distal," which typically refers to that part or component of the instrument that is farther away from the user, refers to the portion of the curved part that is farthest along an axis that follows the curve of the curved part. That is, while an intermediate portion of a curved part may be farther from the user during use, the portion of the curved part that is farthest along its axis is considered "distal."

In disclosed examples, the radius of curvature of both anvil assembly 506 and cartridge assembly 508 is between about 2.54 cm and about 5.08 cm (about 1.00 inches and about 2.00 inches), and in particular, may be approximately 3.56 cm (1.40 inches). The curved jaw members, as compared to straight jaw members, may help facilitate access to lower pelvis regions, e.g., during lower anterior resection ("LAR"). Additionally, the inclusion of curved jaw members may allow increased visualization to a surgical site and may also allow more room for a surgeon to manipulate target tissue or the jaw members themselves with his or her hand.

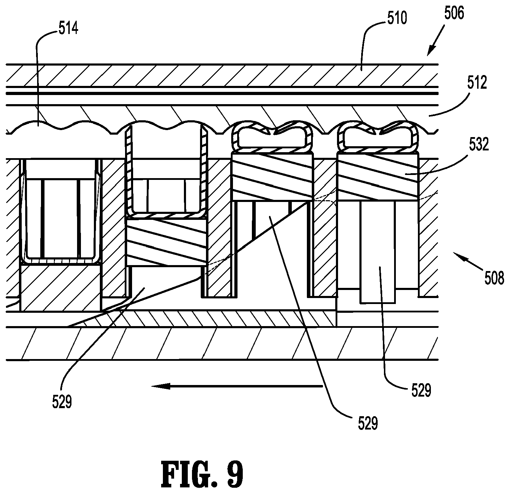

With reference to FIG. 10, anvil assembly 506 includes a longitudinally curved anvil cover 510 and a longitudinally curved anvil plate 512, which includes a plurality of staple forming depressions 514 (FIG. 9). In disclosed examples, the radius of curvature of both anvil cover 510 and anvil plate 512 is between about 2.54 cm and about 5.08 cm (about 1.00 inches and about 2.00 inches) and in particular, may be approximately 3.56 cm (1.40 inches). Anvil plate 512 is secured to an underside of anvil cover to define a channel 511 (FIG. 8) between plate 512 and cover 510. When tool assembly 504 is in the approximated position (FIG. 8), staple forming depressions 514 are positioned in juxtaposed alignment with cartridge assembly 508.

Cartridge assembly 508 includes a longitudinally curved channel or carrier 516 which receives and supports a longitudinally curved cartridge 518. The cartridge 518 can be attached to the channel or carrier by adhesives, a snap-fit connection, or other connection. In disclosed embodiments, the radius of curvature of both carrier 516 and cartridge 518 is between about 2.54 cm and about 5.08 cm (about 1.00 inches and about 2.00 inches), and in particular, may be approximately 3.56 cm (1.40 inches). Cartridge 518 includes a pair of support struts 524 which rest on sidewalls 517 of carrier 516 to stabilize cartridge 518 on carrier 516. Support struts 524 also set the height or location of cartridge 518 with respect to anvil plate 512. An external surface of carrier 516 includes an angled cam surface 516a.

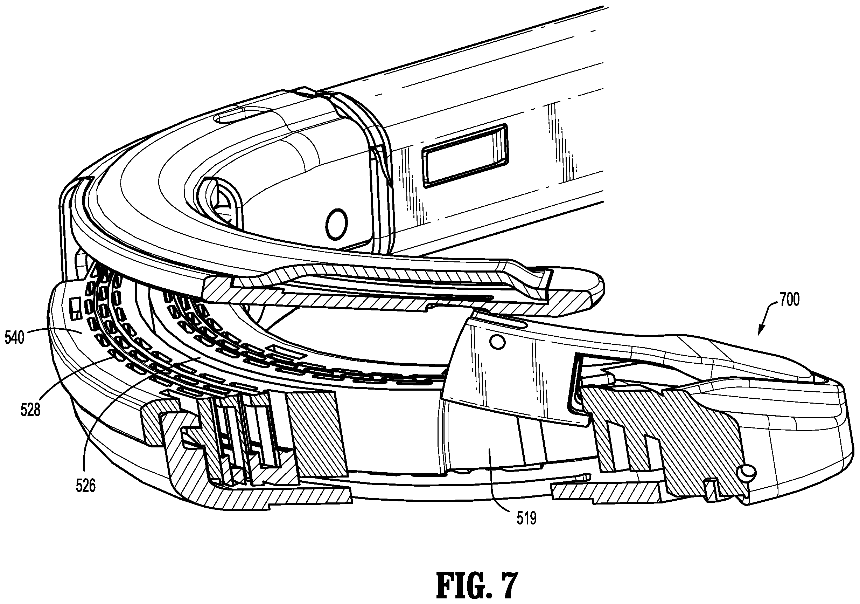

Cartridge 518 defines a plurality of laterally spaced staple retention slots 528, which are configured as holes in tissue contacting surface 540 (see FIG. 7). Each slot 528 is configured to receive a staple 530 therein. Cartridge 518 also defines a plurality of cam wedge slots 529 (see FIG. 9) which accommodate staple pushers 532 and which are open on the bottom (i.e., away from tissue contacting surface 540) to allow a longitudinally curved actuation sled 536 to pass therethrough.

Staple cartridge 518 includes a central longitudinally curved slot 526, and three longitudinally curved rows of staple retention slots 528 positioned on each side of curved longitudinal slot 526 (see FIGS. 7 and 8). In disclosed examples, the radius of curvature of both slot 526 and pusher 532 is between about 2.54 cm and about 5.08 cm (about 1.00 inches and about 2.00 inches), and in particular, may be approximately 3.56 cm (1.40 inches). More specifically, actuation sled 536 passes through cam wedge slots 529 and forces staple pushers 532 towards respective staples 530. The staples are then forced out of their respective staple retention slots 528.

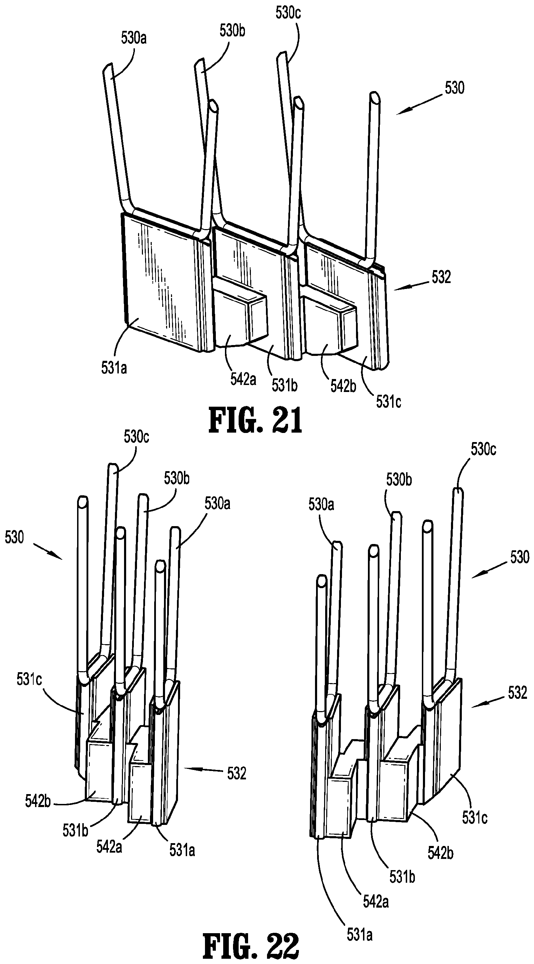

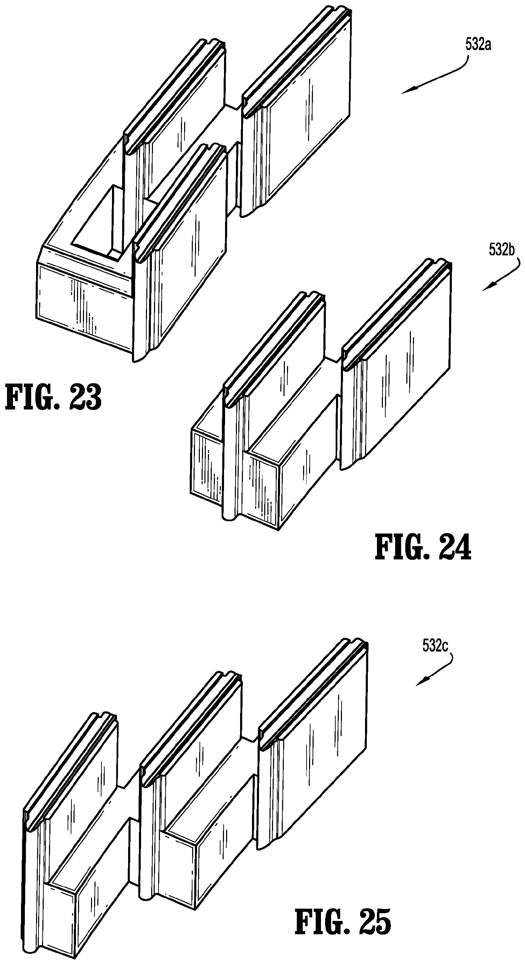

With reference to FIGS. 21 and 22, pushers 532 of the illustrated examples, each engage two or more staples 530. Pushers 532 include a single distally-located triple pusher 532a (FIG. 23), a single proximally-located double pusher 532b (FIG. 24), and a series of triple pushers 532c (one triple pusher 532c is shown in FIG. 25) which extend between double pusher 532b and triple pusher 532a on each side of slot 526. In disclosed examples, portions of pushers 532a, 532b, 532c include various radii of curvature included therewith and are in the range of approximately 2.54 cm to about 3.81 cm (1.00 inches to about 1.50 inches). It is also disclosed that at least one pusher 532a, 532b, 532c includes no curved surfaces - only linearly angled surfaces.

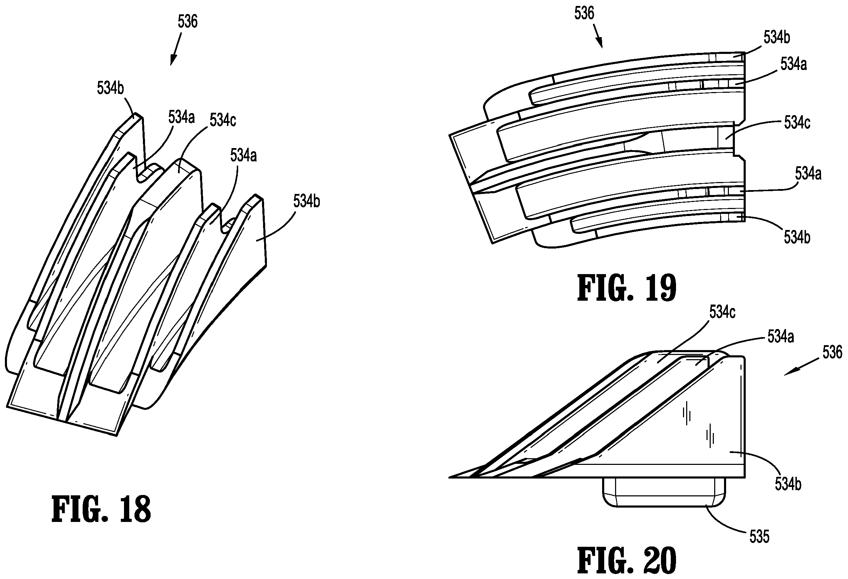

During operation of stapler 10, actuation of its movable handle 22 through successive strokes causes distal advancement of its drive bar 30 (a distal portion of which is illustrated in FIG. 2), such that drive bar 30 pushes a drive assembly 560 through cartridge 518. (Further details of how actuation of movable handle 22 causes distal advancement of drive bar 30 are explained in U.S. Patent No. 6,953,139 to Milliman et al.. The movement of drive assembly 560, and in particular, a dynamic clamping member 606 affixed thereto, moves a longitudinally curved actuation sled 536 (see FIGS. 18-20) through cartridge 518. As sled 536 moves through cartridge 518, longitudinally curved cam wedges 534 of actuation sled 536 sequentially engage pushers 532 to move pushers 532 vertically within staple retention slots 528 and eject staples 530 into staple forming depressions 514 of anvil plate 512. Subsequent to the ejection of staples 530 from retention slots 528 (and into tissue), a cutting edge 606d of dynamic clamping member 606 severs the stapled tissue as cutting edge 606d travels through curved slot 526 of cartridge 518.

Referring to FIG. 8 and in accordance with examples of the present disclosure, cartridge 518 includes a tissue contacting surface 540 including surfaces 540a, 540b, and 540c. Surface 540a is adjacent longitudinal slot 526 and defines a first gap between tissue contacting surface 540 and a bottom surface 544 of anvil plate 512. Surface 540b is located adjacent surface 540a and defines a second gap between tissue contacting surface 540 and bottom surface 544. Surface 540c is located proximal to an outer perimeter of cartridge 518 and defines a third gap between tissue contacting surface 540 and bottom surface 544. The first gap is less than the second gap, which is less than the third gap. When anvil 506 is approximated towards cartridge 508, layers of tissue located between bottom surface 544 and tissue contacting surface 540 are compressed. Since the first gap is the smallest, tissue located between surface 540a and bottom surface 544 is compressed the most. Similarly, the tissue located between surface 540c and bottom surface 544 is compressed the least, with the tissue located between surface 540b and bottom surface 544 being compressed to an intermediate degree. The arrangement of surfaces 540a, 540b, 540c on tissue contacting surface 540 provides a tissue compression gradient extending transverse to a longitudinal axis of the cartridge 518.

Referring to FIGS. 8, 21 and 22 in conjunction with the stepped arrangement of tissue contacting surface 540, the illustrated example of staples 530 include varying leg lengths for cooperating with the varying gaps. Staples 530a have the shortest leg length and are associated with surface 540a. Similarly, staples 530b have an intermediate leg length and are associated with surface 540b, while staples 530c have the longest leg length and are associated with surface 540c. The leg length of staples 530b is between the leg length of staples 530a and 530c. Since the tissue between surface 540a and bottom surface 544 has been compressed the most, the resulting thickness of the tissue is at a minimum, thereby allowing a staple having a shorter leg length (i.e. staple 530a) to be used to join the layers of tissue. The layers of tissue between surface 540b and bottom surface 544 are compressed to an intermediate degree of compression and the resulting thickness of the tissue layers allows a staple having an intermediate leg length (i.e. staple 530b) to be used when joining the layers of tissue. The layers of tissue between surface 540c and bottom surface 544 are compressed the least amount and are thicker than the other layers requiring staples that have the longest leg length (i.e. staples 530c) for joining the layers of tissue.

In particular, the illustrated example of pusher 532 includes plates 531a, 531b, 531c, which cooperate with staples 530a, 530b, 530c, respectively. Plate 531a has a height which is greater than the height of plate 531b. Additionally, the height of plate 531b is greater than the height of plate 531c. Pusher 532 further includes cam members 542 that are longitudinally staggered. As sled 536 translates distally through cartridge 518, cam wedges 534 engage cam members 542 of pusher 532, thereby urging pusher 532 in a direction transverse to the longitudinal axis of cartridge 518 and urging staples 530 towards staple forming depressions 514 of anvil plate 512. In particular, cam wedges 534 are longitudinally staggered such that when they engage staggered cam members 542, the resulting forces applied to move pusher 532 towards tissue contacting surface 540 are evenly applied.

With continued reference to FIGS. 21 and 22, staples 530a, 530b, 530c ride on pusher 532 (for illustrative purposes, pusher 532c from FIG. 25 is shown). Additionally, cam members 542 of each pusher 532 include cam surfaces 542a and 542b. Each cam surface 542a, 542b is configured to be contacted by cam wedges 534. In particular, and with reference to FIGS. 21-25, cam wedges 534a are configured to cam surfaces 542a; cam wedges 534b are configured to engage cam surfaces 542b; central section 534c of sled 536 is configured to travel through slot 526.

Referring to FIG. 20, the illustrated example of actuation sled 536 includes a longitudinally curved projection 535 depending from a lower surface thereof. Projection 535 is configured to travel within a slot 515 (FIG. 10) of channel or carrier 516. In disclosed examples, the radius of curvature of both cam wedges 534 and projection 535 is between about 2.54 cm and about 5.08 cm (about 1.00 inches and about 2.00 inches), and in particular, may be approximately 3.56 cm (1.40 inches).

With reference to FIG. 10, proximal body portion 502 includes an inner body 503 formed from molded half-sections 503a and 503b, a drive assembly 560 and a drive locking assembly 564. Proximal body portion 502 is coupled to tool assembly 504 by a mounting assembly 570. Mounting assembly 570 has a pair of extensions 576 which extend into a proximal end of carrier 516. Each extension 576 has a transverse bore 578 which is aligned with a hole 580 in the cartridge 518 such that mounting assembly 570 is pivotally secured to cartridge 518 by pin 582. Mounting assembly 570 is fixedly secured to half-section 503a by a pair of vertical protrusions 584. Vertical protrusions 584 extend upwardly from mounting assembly 570 and frictionally fit into corresponding recesses (not shown) in half-section 503a.

With continued reference to FIG. 10, the illustrated example of anvil cover 510 includes a proximally extending finger 588 having a pair of cutouts 590 formed therein. Cutouts 590 are positioned on each lateral side of finger 588 to help secure anvil cover 510 to half-section 503a. More particularly, half-section 503a includes a channel 505 therein, and channel 505 includes a pair of protrusions 505a. Finger 588 of anvil cover 510 mechanically engages channel 505 of half-section 503a, such that cutouts 590 are aligned with protrusions 505a. An outer sleeve 602 covers the finger and channel. The configuration of finger 588 and channel 505 facilitates a secure connection between anvil cover 510 and half-section 503a. Moreover, this connection results in a non-movable (e.g., non-pivotable) anvil assembly 506 with respect to proximal body portion 502.

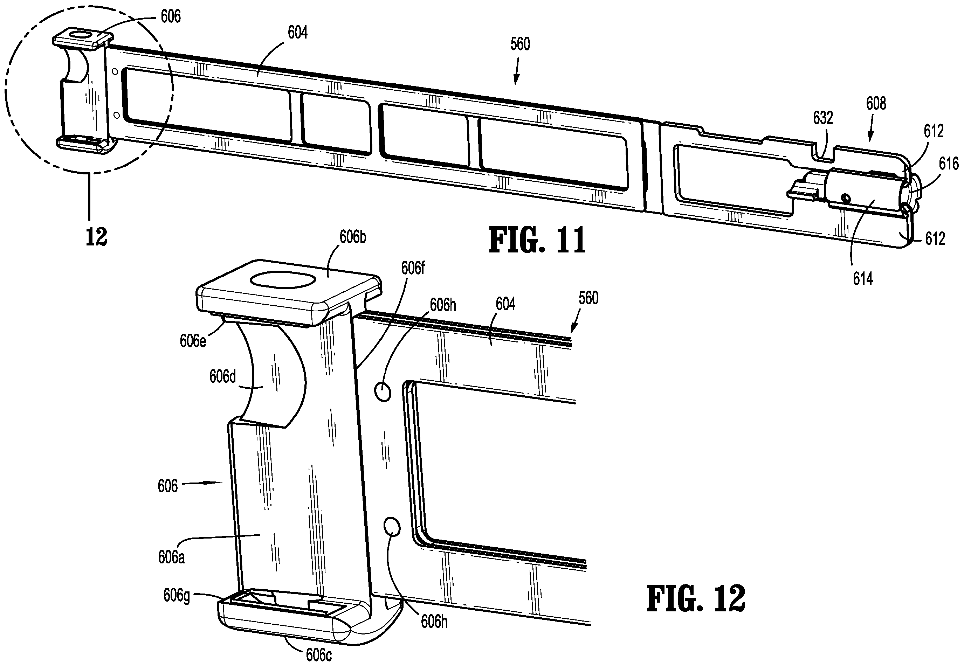

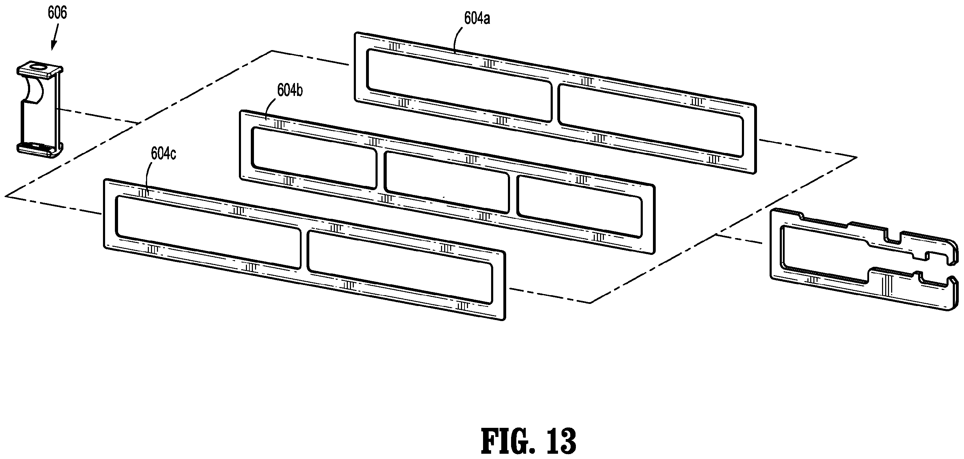

Referring to FIGS. 11-13, drive assembly 560 includes a flexible drive beam 604 which is constructed from three stacked metallic sheets 604 a-c and a proximal engagement portion 608. At least a portion of drive beam 604 is sufficiently flexible to be advanced through the curvature of the tool assembly 504. Drive beam 604 has a distal end which is secured to a dynamic clamping member 606 via a butt weld 606f (FIG. 12). Spot welds 606h, which are configured to hold sheets 604 a-c together, are also shown in FIG. 12.

Engagement section 608 is fastened to a proximal portion of middle sheet 604b (e.g., via a butt weld) and includes a stepped portion defining a shoulder 610. A proximal end of engagement section 608 includes diametrically opposed inwardly extending fingers 612. Fingers 612 engage a hollow drive member 614 to fixedly secure drive member 614 to the proximal end of beam 604. Drive member 614 defines a proximal porthole 616 which receives the distal end of a control rod of drive bar 30 (see FIG. 2) when loading unit 500 is attached to surgical stapling instrument 10.

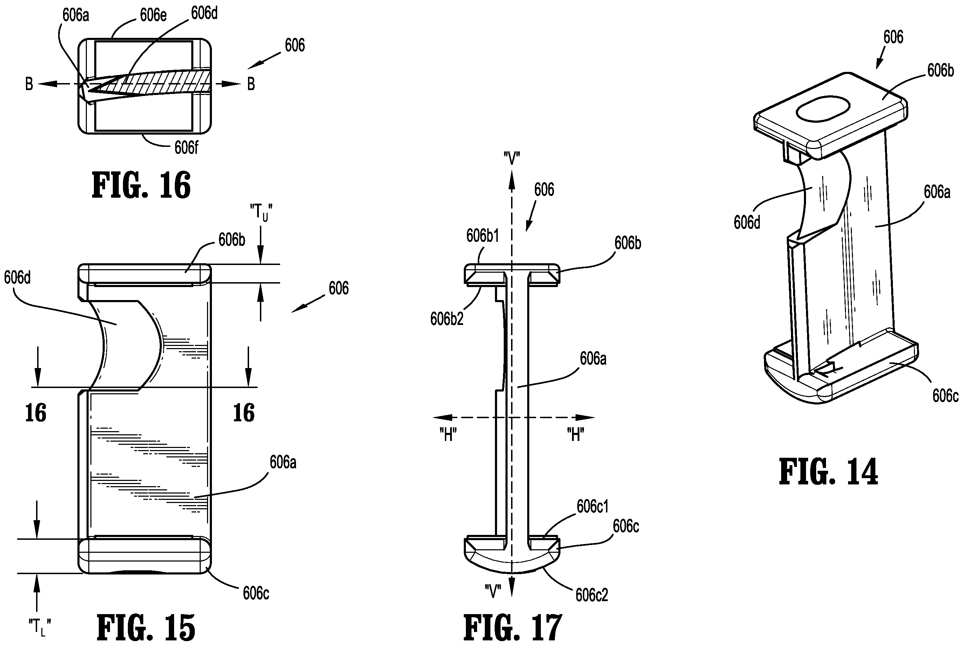

With reference to FIGS. 14-17, dynamic clamping member 606 includes a vertical strut 606a, an upper beam 606b and a lower beam 606c. A knife or cutting edge 606d is formed on a distal face of vertical strut 606a. As illustrated, the width of vertical strut 606a is equal to the width of drive beam 604 of drive assembly 560 (see FIG. 12). With particular reference to FIG. 16, vertical strut 606a and knife 606d are longitudinally curved from a first lateral side 606e of clamping member towards a second lateral side 606f of clamping member 606. Both upper beam 606b and lower beam 606c are linearly disposed with respect to longitudinal axis "A-A."

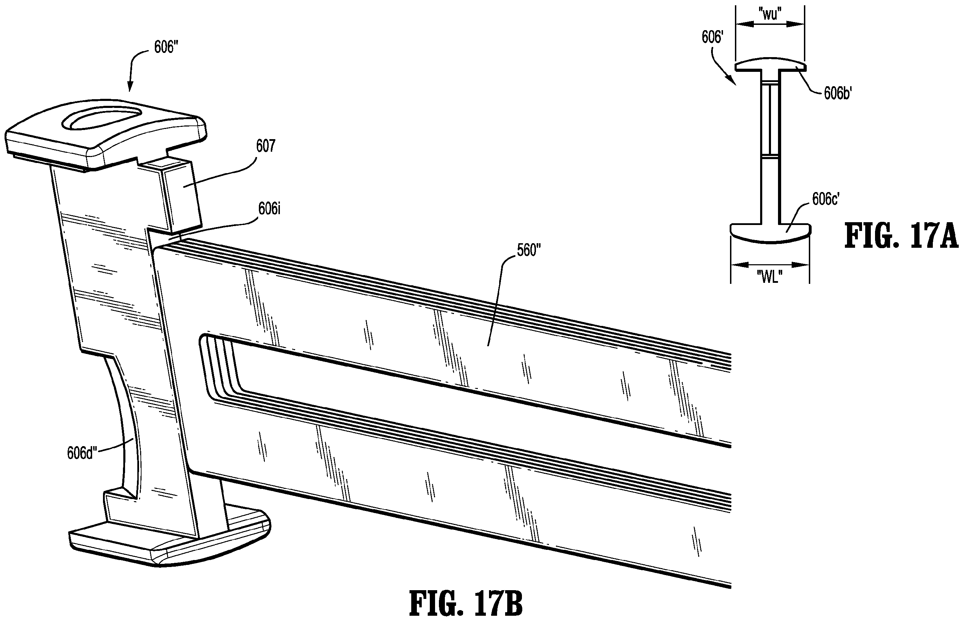

As illustrated in FIGS. 14-17A, the present disclosure includes examples of dynamic clamping member 606 that are asymmetrical. For instance, in the example illustrated in FIGS. 15 and 17, lower beam 606c is thicker than upper beam 606b. In this example, dynamic clamping member 606 is asymmetrical about horizontal axis "H-H" illustrated in FIG. 17. It is envisioned that lower beam 606c includes a thickness "T_{L}", which is between about 0.127 cm and about 0.254 cm (0.050 inches and about 0.100 inches), and in particular, may be approximately 0.172 cm (0.068 inches). It is envisioned that upper beam 606b includes a thickness "T_{U}", which is between about 0.064 cm and about 0.127 cm (0.025 inches and about 0.050 inches), and in particular, is approximately 0.094 cm (0.037 inches).

An additional example of an asymmetrical dynamic clamping member 606 is also illustrated in FIG. 17. In this example, the transverse cross-sectional shape of upper beam 606b includes an upper planar surface 606b1 and a lower planar surface 606b2. The cross-sectional shape of lower beam 606c includes an upper planar surface 606c1 and a lower arcuate surface 606c2. In this example, dynamic clamping member 606 is asymmetrical about the horizontal axis "H-H."

The example shown in FIGS. 16 and 17 illustrates proximal portion of vertical strut 606a being off-center with respect to the remainder of clamping member 606. More particularly, it is envisioned that the center of vertical strut 606a is between about 0.178 cm and about 0.229 cm (0.070 inches and about 0.090 inches) e.g. approximately 0.203 cm (0.080 inches) from first lateral side 606e of clamping member 606, and is between about 2.29 cm (0.90 inches) and about 0.279 cm (0.110 inches) e.g. approximately 0.254 cm (0.100 inches) from second lateral side 606f of clamping member 606. In this example, dynamic clamping member 606 is asymmetrical about vertical axis "V-V" illustrated in FIG. 17.

With reference to FIG. 17A, dynamic clamping member 606' is shown. Lower beam 606c' is wider than upper beam 606b' of dynamic clamping member 606'. More particularly, it is envisioned that a width "wl" of lower beam 606c' is between about 0.457 cm and about 0.508 cm (0.180 inches and about 0.200 inches), and that a width "wu" of upper beam 606b' is between about 0.406 cm and about 0.457 cm (0.160 inches and about 0.180 inches). In this example, dynamic clamping member 606' is asymmetrical about the horizontal axis "H-H." Further, while not explicitly shown, it is envisioned that upper beam 606b' is wider than lower beam 606c' of a dynamic clamping member 606 of the present disclosure. Additionally, dynamic clamping member 606' is shown as being longitudinally linear (vis-à-vis longitudinally curved), in accordance with examples of the present disclosure.

The asymmetrical examples of dynamic clamping member 606 of the present disclosure help ensure proper orientation of dynamic clamping member 606 during assembly of surgical stapling instrument 10 or loading unit 500. That is, the asymmetry of dynamic clamping member 606 prevents dynamic clamping member 606 from improper placement with respect to tool assembly 504, since dynamic clamping member 606 can only physically fit in a particular orientation. In particular, the asymmetry ensures that knife 606d faces distally and is positioned to travel through the space between cartridge assembly 508 and anvil assembly 506, for example.

With reference to FIG. 17B, the present disclosure includes another example of a dynamic clamping member 606" that is also configured to help ensure proper orientation of dynamic clamping member 606" during assembly of surgical stapling instrument 10 or loading unit 500. Dynamic clamping member 606" includes a protrusion 607 extending from a proximal surface 606i thereof. In the illustrated example, a drive assembly 560" has a smaller height than example of drive assembly 560' illustrated in FIGS. 10-13. Protrusion 607 is shown being disposed on a lower portion of dynamic clamping member 606" (i.e., on the opposite side as cutting edge 606d") and to one side of drive assembly 560", but it is envisioned that protrusion 607 is disposed on the other side of drive assembly 560".

As discussed above, the inclusion of protrusion 607 helps ensure proper orientation of dynamic clamping member 606". More particularly, it is envisioned that extensions 576 of mounting assembly 570 would physically prevent further assembly of dynamic clamping member 606" being incorrectly fastened to drive assembly 560" (e.g., when dynamic clamping member 606" is up-side-down with respect to drive assembly 560".

It is further envisioned that dynamic clamping member 606, 606' may include any combination of the asymmetrical features discussed herein and may also include protrusion 607 of dynamic clamping member 606".

With additional reference to dynamic clamping member 606 of FIGS. 14-17A, it is envisioned that each of upper beam 606b and 606c includes a plastic material or layer which is injection molded onto an outwardly facing surface of each beam 606b and 606c. Plastic layer provides reduced frictional engagement between dynamic clamping member 606 and cartridge and anvil assemblies 508 and 506, respectively, during actuation of tool assembly 504.

Referring back to FIG. 8, channel 511 is configured and dimensioned accordingly to accommodate a corresponding example of upper beam 606b of clamping member 606; slot 526 is configured and dimensioned accordingly to accommodate a corresponding example of vertical strut 606a of clamping member 606. As can be appreciated, when used with the example of dynamic clamping member 606 of FIG. 17A, channel 511 is too narrow to accommodate lower beam 606c of dynamic clamping member 606.

With reference to FIG. 10, when drive assembly 560 is advanced distally within tool assembly 504, upper beam 606b moves within channel 511 defined between anvil plate 512 and anvil cover 510, and lower beam 606c moves over an exterior surface of carrier 516. When lower beam 606c engages and moves over cam surface 516a, cartridge assembly 508 pivots from the open position to the closed position. As dynamic clamping member 606 continues to move distally along and through tool assembly 504, the maximum gap between anvil plate 512 and cartridge 518 is defined by engagement of layer 606e on upper beam 606b (FIG. 12) and a lower surface defining channel 511, and engagement of a layer 606g on lower beam 606c with the external surface of carrier 516. In disclosed examples, the height of channel 511 is greater than the height of upper beam 606b, providing clearance between the upper surface of dynamic clamping member 606 and the anvil plate 512 so that upper beam 606b of dynamic clamping member 600 does not simultaneously engage the upper and lower surfaces of anvil channel 511.

With continued reference to FIG. 10, loading unit 500 includes a locking mechanism 564 including a locking member 620 and a locking member actuator 622. Locking member 620 is rotatably supported within a longitudinal or axial slot 625 formed in a proximal portion of an upper housing half 503a of inner body 503 of loading unit 500. Locking member 620 is movable from a first position, in which locking member 620 maintains drive assembly 560 in a prefired position, to a second position in which drive assembly 560 is free to move axially.

Locking member 620 includes a semi-cylindrical body 624 which is slidably positioned within transverse slot 625 formed in upper housing half 503a of body portion 503. Body 624 includes a radially inwardly extending cam member 628 and a radially inwardly extending finger 630. Finger 630 is dimensioned to be received within a notch 632 formed in drive assembly 560. Engagement of finger 630 in notch 632 of drive assembly 560 prevents drive assembly 560 from moving linearly within body portion 503 to prevent actuation of loading unit 500 prior to attachment of loading unit 500 to surgical instrument 10.

Locking member actuator 622 is slidably positioned within axial slot 625 formed in upper housing half section 503a of body portion 503 of loading unit 500. Actuator 622 includes a proximal abutment member 636, a distal spring guide 627, and a central cam slot 640. Axial slot 641 in the housing half section 503a intersects transverse slot 625 such that cam member 628 of locking member 620 is slidably positioned within cam slot 640 of locking member actuator 622. A biasing member or spring 642 is positioned about spring guide 627 between a distal surface of actuator 622 and a wall 641a defining the distal end of axial slot 641. Spring 642 urges actuator 622 to a first position within axial slot 641. In the first position, abutment member 636 is positioned on insertion tip 650 of proximal body portion 502 (FIG. 3) and cam slot 640 is positioned to locate cam member 628 such that finger 630 of lock member 620 is positioned within notch 632 of drive assembly 560.

Prior to attachment of loading unit 500 onto surgical instrument 10, spring 642 urges actuator 622 to the first position to maintain the lock member 620 in its first position as discussed above. When insertion tip 650 of loading unit 500 is linearly inserted into the open end of the body portion 18 (FIG. 2) of surgical instrument 10, nubs 652 of insertion tip 650 (FIG. 3) move linearly through slots (not shown) formed in open end of body portion 18. As nubs 652 pass through the slots, the proximal end of abutment member 636, which is angularly offset from nubs 652, abuts a wall defining the slots for receiving nubs. As loading unit 500 is moved farther into body portion, locking member actuator 622 is moved from its first position to its second position. As actuator 622 is moved to its second position, lock member 620 is cammed from its first position engaged with notch 632 of drive assembly 560 to its second position to move finger 630 from notch 632. The locking mechanism including locking member 620 and locking member actuator 622 prevents advancement of the drive assembly 560 of loading unit 500 prior to loading of loading unit 500 onto a surgical instrument 10.

In the examples illustrated in FIGS. 3 and 10, locking member actuator 622 includes an articulation lock portion 637 disposed thereon. In particular, articulation lock portion 637 extends in an approximate right angle from abutment member 636. Articulation lock portion 637 is configured to physically prevent the longitudinal translation of an articulation member (not shown) of a handle portion of a surgical instrument having articulation capabilities. That is, even when loading unit 500 is engaged with a surgical instrument 10 that is otherwise capable of articulation (i.e., pivotable movement of the jaw members with respect to the elongated portion 18), articulation lock portion 637 of loading unit 500 prevents an articulation member from entering loading unit 500.

Referring to FIG. 10, upper half-section 503a of proximal body portion 502 defines a longitudinal slot 660 which receives a leaf spring 662. Leaf spring 662 is confined within slot 660 by outer sleeve 602. Leaf spring 662 has an angled proximal end 664 which is positioned to abut shoulder 610 (FIG. 11) of engagement section 608 of drive beam 604 when drive beam 604 is in its retracted position. When drive beam 604 is advanced distally by advancing drive bar 30, as described above, leaf spring 662 is flexed upwardly by shoulder 610 of drive beam 604 to permit distal movement of drive beam 604.

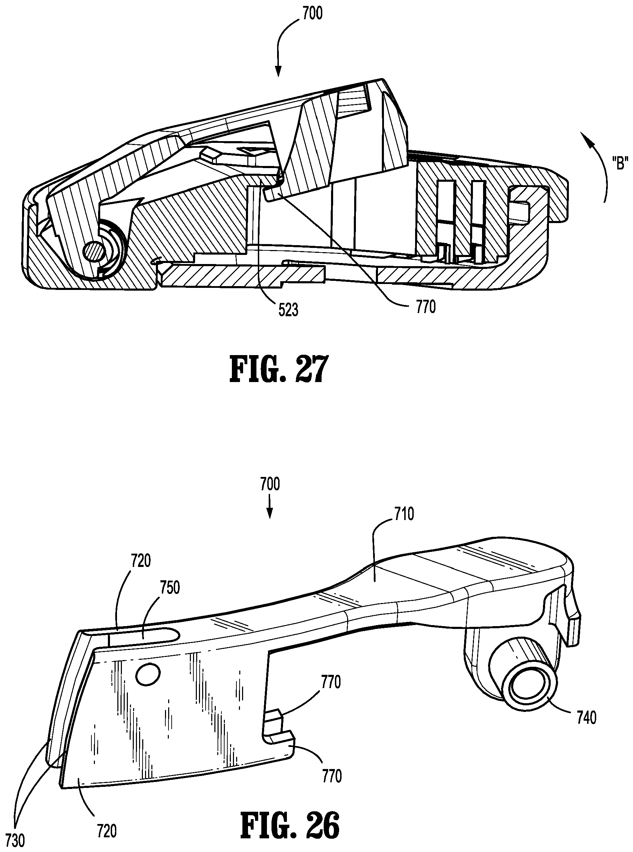

Referring to FIGS. 4, 7, and 26-30, loading unit 500 also includes a tissue stop 700. Tissue stop 700 includes a body 710, a pair of legs 720 extending proximally from the body 710, a stopping portion 730, a pair of laterally opposed protrusions 740 extending transversely from body 710 (See FIG. 26), and a knife channel 750 disposed between pair of legs 720. Tissue stop 700 is pivotally connected to a distal portion of cartridge assembly 508 via the engagement between protrusions 740 and a corresponding pair of apertures (not shown) disposed within cartridge assembly 508. Cartridge assembly 508 includes an opening 519 (FIGS. 7 and 10) adapted to receive both legs 720 of tissue stop 700. A recess 521 is positioned distally of opening 519 and is adapted to receive a portion of tissue stop 700 therein. The recess 521 and opening 519 are shown in FIG. 10.

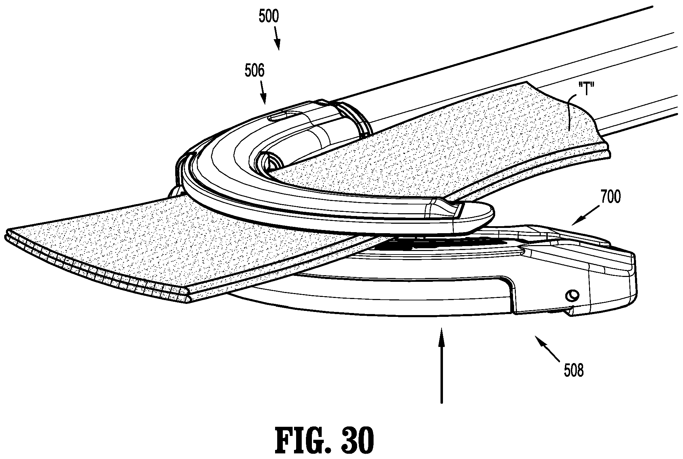

Tissue stop 700 is movable between a first position (FIG. 4), which corresponds to when the jaw members are in an open position where an upper surface 701 thereof is disposed between cartridge assembly 508 and anvil assembly 506 (FIG. 4 illustrates the jaw members in a partially approximated position; FIG. 6 illustrates the jaw members in a fully opened position), and a second position (FIG. 30), which corresponds to when the jaw members are in the approximated position and where upper surface 701 of tissue stop 700 is substantially flush with tissue contacting surface 514 of cartridge 518. (In FIG. 30, upper surface 701 is hidden as upper surface 701 is within cartridge assembly 508.) A biasing member 760 (FIG. 10), a portion of which is disposed around protrusion 740, urges tissue stop 700 towards its first position. Tissue stop 700 also includes a finger 770 (FIG. 26) extending distally from each leg 720. With specific reference to FIG. 27, when the jaw members are in the open position, fingers 770 of tissue stop 700 engage a lip 523 disposed on cartridge assembly 508 to limit the amount of movement imparted by biasing member 760 in the general direction of arrow "B" in FIG. 27.

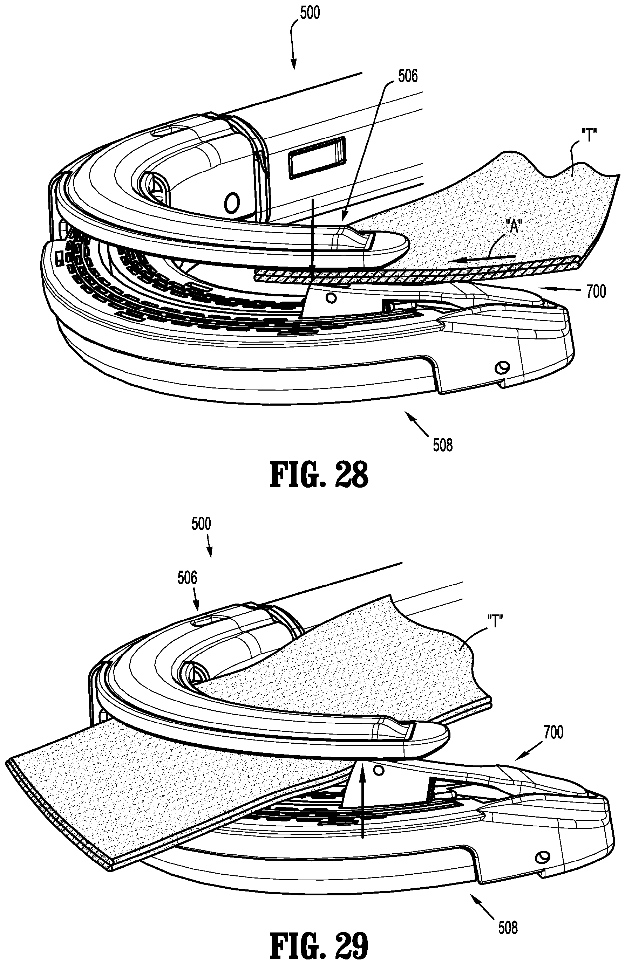

When tissue stop 700 is in its first position, tissue "T" is proximally insertable (in the general direction of arrow "A" in FIG. 28) from distally beyond tissue stop 700, to a location that is between anvil assembly 206 and cartridge assembly 508 and proximal of tissue stop 700 (see FIGS. 28 and 29). In this position, stopping portion 730, which is disposed at an oblique angle (e.g., between about 45° and about 90°) with respect to tissue contacting 540 of cartridge assembly 508, impedes tissue from distally escaping the tool assembly 504. When the jaw members are approximated (e.g., when cartridge assembly 508 is pivoted towards anvil assembly 506), tissue stop 700 (or tissue "T") contacts anvil assembly 506, thus causing tissue stop 700 to pivot from its first position towards its second position. Legs 720 of tissue stop 700 are configured to lie within opening 519 (i.e., equal to or below the tissue contacting surface 540) of cartridge assembly 508 when tissue stop 700 is in its second position, such that legs 720 do not interfere with the location of the tissue with respect to the cartridge assembly 508 and respect to anvil assembly 506 (i.e., so that the staples can be deployed into tissue lying over the tissue stop). When the cartridge assembly 508 moves away from anvil assembly 506, tissue stop 700, under the influence of biasing member 760, returns to its first position.

With additional regard to knife channel 750, knife channel 750 is configured to allow vertical strut 606a (including cutting edge 606d) of dynamic clamping member 606 to travel distally past a portion of tissue stop 700 (i.e., at least to a location adjacent the distal-most longitudinal slot 528). Additionally, it is envisioned that at least a portion of knife channel 750 (e.g., the portion that is contacted by cutting edge 606d) is over molded with plastic or another suitable material.

While not explicitly illustrated, it is also envisioned that tissue stop 700 is usable with a surgical instrument having parallel jaws and/or an electrosurgical instrument. An example of a surgical instrument having parallel jaws is described in commonly-owned U.S. Pat. No. 7,237,708 to Guy et al.. An example of an electrosurgical instrument is described in commonly-owned Patent Application No. 10/369,894, filed on February 20, 2003, entitled VESSEL SEALER AND DIVIDER AND METHOD OF MANUFACTURING THE SAME.

The present disclosure also relates to methods of using the described surgical instrument 10 or loading unit 500 to perform a lower anterior resection. Such a method includes providing surgical instrument 10 or loading unit 500, positioning jaw members adjacent tissue, approximating one jaw member (e.g., cartridge assembly 508) with respect to the other jaw member (e.g., anvil assembly 506), advancing drive assembly 560 such that dynamic clamping member 606 and at least a portion of drive assembly 560 move along a curvilinear path to cause staples 530 to be ejected into tissue "T" and to cut tissue "T." In certain examples, the jaw members are approximated, and the interior of the intestinal tissue is then washed out or otherwise cleansed. The tissue is then cut and stapled. In this way, the interior intestinal tissue is cleansed up to the location of the jaw members.

The present disclosure also relates to methods of assembling surgical instrument 10 or loading unit 500. Such a method includes positioning asymmetrical dynamic clamping member 606, 606' in mechanical engagement with a portion of tool assembly 504, and wherein the positioning step automatically results in the proper positioning of asymmetrical dynamic clamping member 606. Another method includes attaching dynamic clamping member 606" to drive assembly 560" in a way that would enable fail-safe positioning of dynamic clamping member 606" with respect to tool assembly 504.

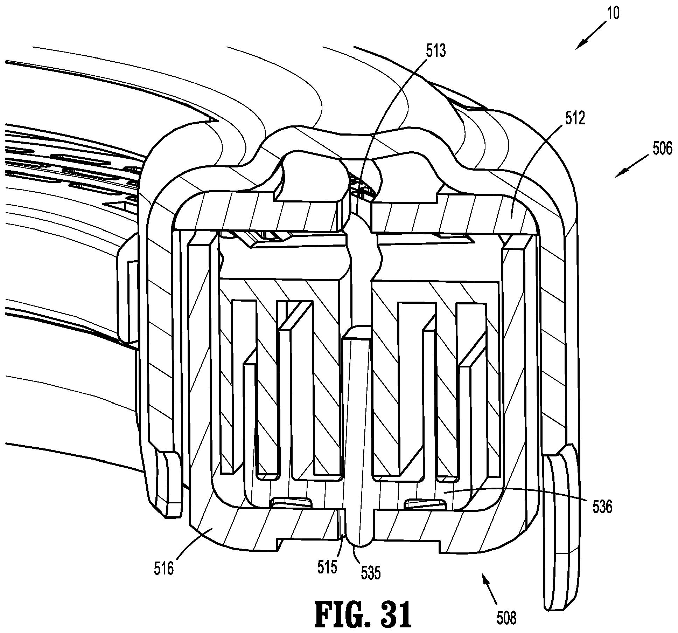

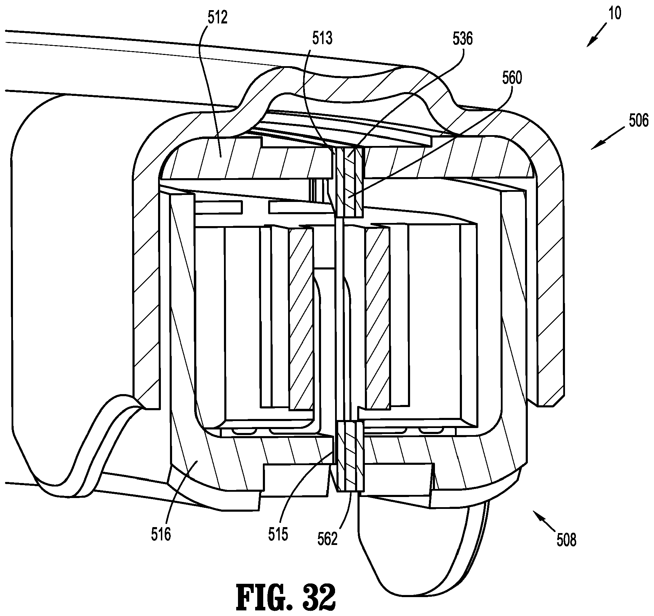

Other features of the present disclosure are shown in the cross-sectional views of FIGS. 31 - 32. Surgical instrument 10 includes the actuation sled 536 (FIG. 31) and drive assembly 560 (FIG. 32).

With particular reference to FIG. 31, a transverse cross-sectional view of surgical instrument 10 (e.g., loading unit) taken along a portion of actuation sled 536 is shown. The jaw members of surgical instrument 10 are shown and include an anvil assembly 506 and a cartridge assembly 508, which includes a channel or carrier 516. Here, actuation sled 536 includes a projection 535 depending from a lower surface thereof. (FIG. 20 also illustrates actuation sled 536 having projection 535 depending from a lower surface thereof.) Projection 535 is configured to travel within a slot 515 of a carrier 516. As actuation sled 536 is translated distally, projection 535 helps ensure that actuation sled 536 follows the curvature of the jaw members.

With particular reference to FIG. 32, a transverse cross-sectional view of surgical instrument 10 taken along a portion of drive assembly 560 is shown. Here, drive assembly 560 includes a lower portion 562 that is configured to travel within slot 515 of carrier 516. Additionally, an upper portion 563 of drive assembly 560 is configured to travel with a slot 513 (see also FIG. 31, for example) in anvil plate 512. For example, the drive beam 604 extends into the slot 515 and may also extend into slot 513. Upon distal translation of drive assembly 560, the interaction between lower portion 562 and upper portion 563 of drive assembly 560 with slots 515 and 513, respectively, helps ensure that drive assembly 560 follows the curvature of the jaw members. It is also envisioned and within the scope of the present disclosure that drive assembly 560 only engages a single slot 513 or 515. As noted above, these structures can be incorporated in a surgical instrument that does not have a loading unit incorporating the jaws of the instrument in a replaceable assembly and in which the staple cartridge is removable and/or reloadable.

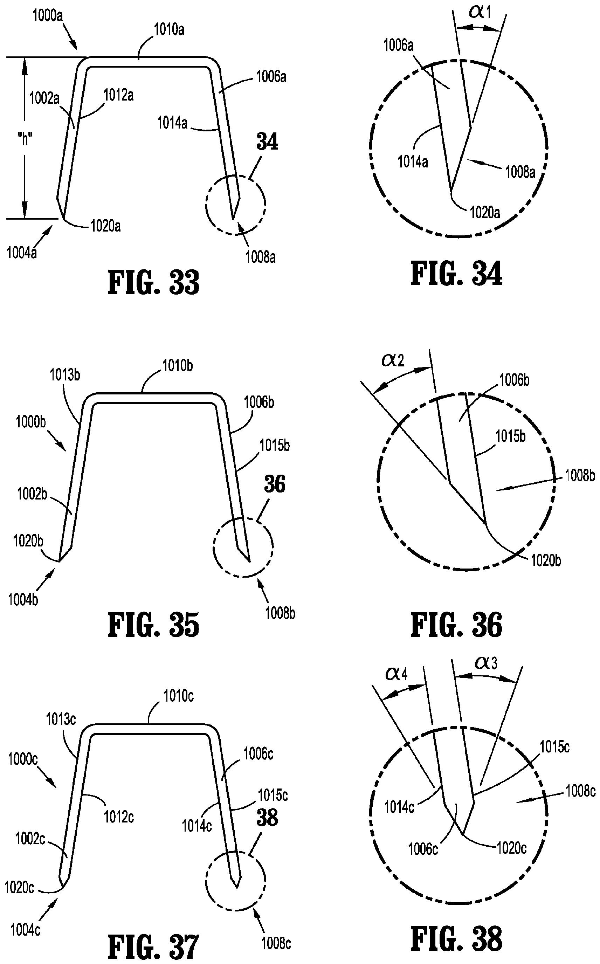

With reference to Figures 33-38, various configurations of a fastener or staple 1000a-c for use with surgical stapling instrument 10 and loading unit 500 are illustrated. In particular, Figures 33 and 34 illustrate a staple 1000a having a first configuration, Figures 35 and 36 illustrate a staple 1000b having a second configuration, and Figures 37 and 38 illustrate a staple 1000c having a third configuration. It envisioned that each configuration of staples 1000a-c facilitates proper formation of staples 1000a-c after staples 1000a-c have been ejected from cartridge assembly 508 in certain circumstances.

Each staple 1000a-c respectively includes a first staple leg 1002a-c having a first staple tip 1004a-c, a second staple leg 1006a-c having a second staple tip 1008a-c, and a backspan 1010a-c interconnecting first staple leg 1002a-c and second staple leg 1006a-c, respectively. Additionally, each staple 1000a-c includes a cross-section that is either circular, rectangular, or any other regular or irregular shape along at least a majority of its length. Further, each staple 1000a-c may be formed from a wire having the same cross-section of the resulting staple 1000a-c.

With particular reference to Figures 33 and 34, first and second staple tips 1004a and 1008a each define an angle α1, with an inner portion or edge 1012a, 1014a of each respective leg 1002a, 1004a including a staple point 1020a aligned therewith. It is envisioned that α1 is between about 25° and about 35°, e.g., equal to about 30°.

With particular reference to Figures 35 and 36, first and second staple tips 1004b and 1008b each define an angle α2, with an outer portion or edge 1013b, 1015b of respective legs 1002b, 1004b including a staple point 1020b aligned therewith. It is envisioned that α2 is between about 25° and about 35°, e.g., equal to about 30°.

With particular reference to Figures 37 and 38, first and second staple tips 1004c and 1008c each define two angles α3 and α4. In this example where first and second staple tips 1004c and 1008c each define two angles α3 and α4, staple tips 1004c and 1008c are chisel-like. A staple point 1020c of each staple leg 1002 and 1006c is disposed between an extension of inner portion or edge 1012c, 1014c and an extension of outer portion or edge 1013c, 1015c of respective legs 1002c, 1006c. It is envisioned that each of α3 and α4 is between about 20° and about 50° (e.g., between about 25° and about 35°, between about 40° and about 50°, or equal to about 45°). It is further envisioned that α3 is larger than α4, that α3 is smaller than α4, and that α3 is equal to or substantially equal to α4. In the examples where α3 is equal to or substantially equal to α4, it is envisioned that staple point 1020c of each staple leg 1002c and 1006c is centered or off-centered with regard to extension of outer portion or edge 1013c, 1015c of respective legs 1002c, 1006c.

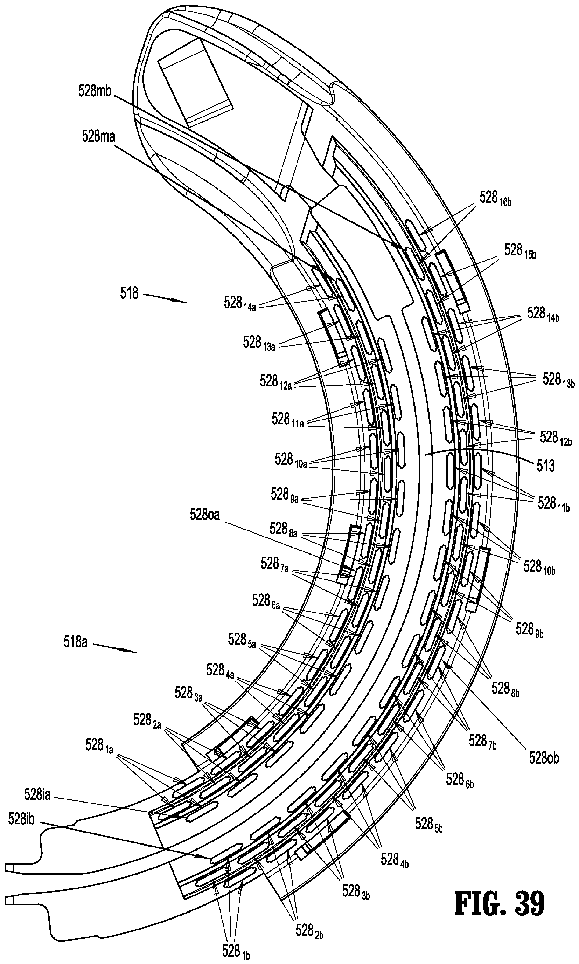

With particular reference to Figures 10 and 39, according to the invention, it is envisioned that some staples 530 are different sizes from other staples 530 within cartridge 518. For instance, it is envisioned that the height "h" (see FIG. 33) of certain staples 530 is about 3.5 mm and/or about 4.0 mm and the height of other staples is about 4.5 mm and/or about 5.0 mm. More particularly, it is disclosed that the staples 530 in a proximal portion 518a (e.g., staple retention slots 528₁ₐ-528₅ₐ and 528_{1b}-528_{5b}) of cartridge 518 are about 4.0 mm, while the staples 530 in the other portions of cartridge 518 are about 4.5 mm and/or about 5.0 mm. It is further envisioned that the shorter (e.g., 4.0 mm) staples 530 are only included within staple retention slots 528 in outer-most rows 528ₒₐ and 528_{ob} with respect to slot 513 (e.g., staple retention slots 528₁ₐ-528₅ₐ and 528_{1b}-528_{5b} therein) and/or in middle rows 528ₘₐ and 528_{mb} (e.g., staple retention slots 528₁ₐ-528₅ₐ and 528_{1b}-528_{5b} therein) (i.e., not within any staple retention slots 528 in inner rows 528ᵢₐ and 528_{ib}) of cartridge 518 (see Figures 8 and 39). It is further envisioned that the shorter staples 530 (e.g., 4.0 mm) are included on an inner side of curvature of slot 513 (e.g., staple retention slots 528₁ₐ-528₅ₐ therein) and the longer staples 530 (e.g., about 4.5 mm and/or about 5.0 mm) are included on an outer side of curvature of slot 513 (e.g., 528_{1b} - 528_{16b}).

It is further envisioned that shorter (e.g., 4.0 mm) staples 530 are included within staple retention slots 528₁ₐ - 528₁₄ₐ in outer row 528ₒₐ, within staple retention slots 528₁ₐ - 528₁₄ₐ in middle row 528ₘₐ, within staple retention slots 528₁ₐ - 528₁₂ₐ in inner row 528ᵢₐ, and within staple retention slots 528_{1b} - 528_{14b} in inner row 528_{ib}; medium (e.g.,. 4.5 mm) staples 530 are included within staple retention slots 528_{1b} - 528_{16b} in middle row 528_{mb}; and large (e.g., 5.0 mm) staples 530 are included within staple retention slots 528_{1b}-528_{16b} in outer row 528_{ob}. That is, in this embodiment, staples 530 within all the rows of retention slots 528 on the inner side of the curvature of slot 513 are all relatively short (e.g., 4.0 mm), staples 530 within the inner row of retention slots 528 on the outer side of the curvature of slot 513 are also relatively short (e.g., 4.0 mm), staples 530 within the middle row of retention slots 528 on the outer side of the curvature of slot 513 are relatively medium (e.g., 4.5 mm), and staples 530 within the outer row of retention slots 528 on the outer side of the curvature of slot 513 are relatively large (e.g., 5.0 mm).

It is contemplated that the size of the staples can be varied according to the shape of the staple line. As discussed above, for example, the staple cartridge 518 has staples of various sizes arranged in the cartridge in a configuration. (See FIG. 39). The staples are arranged in rows on either side of the knife slot 513, and the cartridge 518, as well as the rows of staples and staple retention slots, are curved. There are rows of staples and retention slots on an inner side of the curved knife slot 513, and rows of staples and retention slots on an outer side of the curved knife slot 513. The staples in the rows on the inner side of the curved knife slot can have a different configuration than the staples in the rows on the outer side of the curved knife slot. In the example discussed above, the staples in slots 528₁ₐ through 528₁₄ₐ (on the inner side) can have a different size than the staples in slots 528_{1b} through 528_{16b} (on the outer side). In the example shown, there are three rows on the inner side, and three rows on the outer side. The size of the staples in the various rows can vary, while the configuration on the inner side is different than the configuration for the outer side.

The rows of staple slots and staples can have a proximal portion and a distal portion. The staples in the retention slots on the inner side of the curved knife slot can have a different configuration than the staples in the retention slots on the inner side of the curved knife slot. For example, it is contemplated that the staples in slots 528₁ₐ through 528₁₄ₐ (on the inner side) can have a preselected size, whereas the staples in slots 528_{1b} through 528_{16b} (on the outer side) can have different sizes in each of the rows. Thus, an example of this would be that the staples in all of the retention slots on the inner side are 4.0 mm staples, whereas the staples in the retention slots on the outer side are 4.0 mm, 4.5 mm, and 5.0 mm.

In another example, the staples in all of the retention slots on the inner side are 4.0 mm 4.0 and 3.5 staples, whereas the staples in the retention slots on the outer side are 3.5 mm, 4.0 mm, and 4.5 mm.

In a further example, it is contemplated that the staples in the retention slots in the rows closest to the knife slot 513 are 4.0 mm staples, the staples in the retention slots in the rows farthest from the knife slot are 5.0 mm staples, and the staples in the retention slots of the middle rows (in between those closest to the knife slot and those farthest from the knife slot) are 4.5 mm staples, with the exception that the staples in slots 528**₁ₐ** through 528**₅ₐ** are all 4.0 mm staples. It is contemplated that fewer or less of the staple slots on the inner side of the curved knife slot can be varied in such a manner.

It is also contemplated that the configuration of the staples can be varied in a different sense. That is, the diameter (or width) of the wire used to make the staples can be varied, or they can be made from different materials.

Additionally, in accordance with the invention, some staples 530 are different sizes from other staples 530 within cartridge 518, it is envisioned that cartridge 518 can include staples 530, 1000a, 1000b and/or 1000c therein. It is envisioned that having some staples 530, 1000a-c that are different sizes from other staples 530, 1000a-c within cartridge 518 further facilitates proper formation of the staples after the staples have been ejected from cartridge assembly 508 in certain circumstances. In particular, it is envisioned that a single cartridge 518 includes 4.0 mm staples having a single-angle tip (e.g., staple 1000a of Figures 33 and 34, and staple 1000b of Figures 35 and 36), and 4.5 mm and/or 5.0 mm staples having a double-angle tip (e.g., staple 1000c of Figures 37 and 38).

While the above description contains many specifics, these specifics should not be construed as limitations on the scope of the present disclosure, but merely as illustrations of various embodiments thereof. Therefore, the above description should not be construed as limiting, but merely as exemplifications of various embodiments and non claimed examples. Those skilled in the art will envision other modifications within the scope of the claims appended hereto.

The invention and some non claimed examples may be described with reference to the following numbered paragraphs:-
1. A surgical instrument for surgically joining tissue comprising:
   a handle assembly;
   an endoscopic portion extending distally from the handle assembly and defining a longitudinal axis;
   a pair of jaw members disposed adjacent a distal end of the endoscopic portion and extending generally distally therefrom, each of the jaw members being longitudinally curved with respect to the longitudinal axis, at least one of the jaw members being movable with respect to the other between an open position and an approximated position for engaging body tissue therebetween, the pair of jaw members including a first jaw member and a second jaw member; and
   a plurality of staples disposed at least partially within the second jaw member, wherein each of the staples includes a pair of legs depending from a backspan, wherein each leg includes a staple tip defining a first angle α, and wherein the first angle α1 is between about 25° and about 35°.
2. The surgical instrument according to Paragraph 1, wherein the first angle α1 is approximately equal to 30°.
3. The surgical instrument according to Paragraph 1, wherein each staple tip includes a single staple point.
4. The surgical instrument according to Paragraph 3, wherein each staple point is aligned with an inner edge of the respective staple leg.
5. The surgical instrument according to Paragraph 3, wherein each staple point is aligned with an outer edge of the respective staple leg.
6. The surgical instrument according to Paragraph 1, wherein each staple tip defines a second angle α2, and wherein the second angle α2 is between about 25° and about 35°.
7. The surgical instrument according to Paragraph 1, wherein at least some of the staples disposed within a proximal portion of the second jaw member include a smaller height than at least some of the staples disposed distally of the staples disposed within the proximal portion of the second jaw member.
8. A surgical instrument for surgically joining tissue comprising:
   a handle assembly;
   an endoscopic portion extending distally from the handle assembly and defining a longitudinal axis;
   a pair of jaw members disposed adjacent a distal end of the endoscopic portion and extending generally distally therefrom, each of the jaw members being longitudinally curved with respect to the longitudinal axis, at least one of the jaw members being movable with respect to the other between an open position and an approximated position for engaging body tissue therebetween, the pair of jaw members including a first jaw member and a second jaw member; and
   a plurality of staples disposed at least partially within the second jaw member, wherein each of the staples includes a pair of legs depending from a backspan, wherein each leg includes a staple tip defining a first angle α1 and a second angle α2.
9. The surgical instrument according to Paragraph 8, wherein each staple tip includes a single staple point.
10. The surgical instrument according to Paragraph 9, wherein each staple point is disposed between an extension of an inner edge and an outer edge of the respective staple leg.
11. The surgical instrument according to Paragraph 8, wherein the first angle α1 is between about 20° and 50°.
12. The surgical instrument according to Paragraph 11, wherein the second angle α2 is between about 20° and 50°.
13. The surgical instrument according to Paragraph 8, wherein the first angle α1 is between about 25° and 35°.
14. The surgical instrument according to Paragraph 8, wherein the first angle α1 and the second angle α2 are approximately equal to each other.
15. The surgical instrument according to Paragraph 8, wherein the first angle α1 and the second angle α2 are different from each other.
16. The surgical instrument according to Paragraph 8, wherein at least some of the staples disposed in a proximal portion of the second jaw member include a smaller height than at least some of the staples disposed distally of the staples disposed in the proximal portion of the second jaw member.
17. A loading unit for use with a surgical instrument, the loading unit comprising:
   a proximal body portion configured to engage a portion of a surgical instrument and defining a longitudinal axis;
   a pair of jaw members disposed adjacent the proximal body portion and extending generally distally therefrom, at least one of the jaw members being movable with respect to the other between an open position and an approximated position for engaging body tissue therebetween, the pair of jaw members including a first jaw member and a second jaw member; and
   a plurality of staples disposed at least partially within the second jaw member, wherein at least some of the staples disposed in a first portion of the second jaw member having a smaller height than at least some of the staples disposed in a second portion of the second jaw member.
18. The loading unit according to Paragraph 17, wherein the first portion of the second jaw member is disposed farther proximally than the second portion of the second jaw member.
19. The loading unit according to Paragraph 17, wherein the second jaw member further comprises a slot configured to allow a knife to travel at least partially therealong.
20. The loading unit according to Paragraph 19, wherein the first portion of the second jaw member is disposed on a first lateral side of the slot, and wherein the second portion of the second jaw member is disposed on a second lateral side of the slot.
21. The loading unit according to Paragraph 19, wherein the slot is curved with respect to the longitudinal axis.
22. The loading unit according to Paragraph 21, wherein the first portion of the second jaw member is disposed on an inner side of the curvature of the slot, and wherein the second portion of the second jaw member is disposed on an outer side of the curvature of the slot.
23. The loading unit according to Paragraph 19, wherein each lateral side of the slot of the second jaw member includes an outer row of staple retention slots, an inner row of staple retention slots and a middle row of staple retention slots, wherein the inner row of staple retention slots is closest to the slot, and wherein the first portion of the second jaw member includes the outer rows of staple retention slots and the middle rows of staple retention slots, and wherein the second portion of the second jaw member includes the inner rows of staple retention slots.
24. The loading unit according to Paragraph 21, wherein each lateral side of the slot of the second jaw member includes an outer row of staple retention slots, an inner row of staple retention slots and a middle row of staple retention slots, wherein the inner row of staple retention slots is closest to the slot, wherein the first portion of the second jaw member includes the outer row of staple retention slots, the middle row of staple retention slots and the inner row of staple retention slots on an inside portion of the curvature of the slot, wherein the first portion of the second jaw member includes the inner row of staple retention slots on an outside portion of the curvature of the slot, and wherein the second portion of the second jaw member includes the middle row of staple retention slots and the outer row of staple retention slots on the outside portion of the curvature of the slot.
25. The loading unit according to Paragraph 24, wherein the staples disposed in the outer row of staple retention slots on the outside portion of the curvature of the slot are larger than the staples disposed in the middle row of staple retention slots on the outside portion of the curvature of the slot.
26. The loading unit according to Paragraph 17, wherein at least some of the staples include a pair of legs depending from a backspan, wherein each leg includes a staple tip defining a first angle α1, and wherein the first angle α1 is between about 25° and about 35°.
27. The loading unit according to Paragraph 17, wherein at least some of the staples include a pair of legs depending from a backspan, wherein each leg includes a staple tip defining a first angle α1 and a second angle α2.

## Claims

1. A loading unit (500) for use with a surgical instrument, the loading unit comprising:
a proximal body portion (502) configured to engage a portion of a surgical instrument and defining a longitudinal axis;
a pair of jaw members (506, 508) disposed adjacent the proximal body portion and extending generally distally therefrom, at least one of the jaw members being movable with respect to the other between an open position and an approximated position for engaging body tissue therebetween, the pair of jaw members including a first jaw member and a second jaw member; and
a plurality of staples (530) disposed at least partially within the second jaw member, wherein at least some of the staples disposed in a first portion of the second jaw member having a smaller height than at least some of the staples disposed in a second portion of the second jaw member;
wherein the second jaw member further comprises a slot (513) configured to allow a knife to travel at least partially therealong, wherein the slot is curved with respect to the longitudinal axis;
**characterised in that** the first portion of the second jaw member is disposed (i) on an inner side of the curvature of the slot and (ii) farther proximally than the second portion of the second jaw member, and the second portion of the second jaw member is further disposed on an outer side of the curvature of the slot.

2. The loading unit according to Claim 1, wherein each lateral side of the slot of the second jaw member includes an outer row of staple retention slots (528oa, 528ob), an inner row of staple retention slots (528ia, 528ib) and a middle row of staple retention slots (528ma, 528mb), wherein the inner row of staple retention slots is closest to the slot, wherein the first portion of the second jaw member includes the outer row of staple retention slots (528oa), the middle row of staple retention slots (528ma) and the inner row of staple retention slots (528ia) on an inside portion of the curvature of the slot, wherein the first portion of the second jaw member includes the inner row of staple retention slots (528ib) on an outside portion of the curvature of the slot, and wherein the second portion of the second jaw member includes the middle row of staple retention slots (528mb) and the outer row of staple retention slots (528ob) on the outside portion of the curvature of the slot.

3. The loading unit according to claim 2, wherein the staples disposed in the outer row of staple retention slots (528ob) on the outside portion of the curvature of the slot are larger than the staples disposed in the middle row of staple retention slots (528mb) on the outside portion of the curvature of the slot.

4. The loading unit according to claim 1, wherein each lateral side of the slot of the second jaw member includes an outer row of staple retention slots (528oa, 528ob), an inner row of staple retention slots (528ia, 528ib) and a middle row of staple retention slots (528ma, 528mb), wherein the inner row of staple retention slots is closest to the slot; wherein the first portion of the second jaw member includes the inner rows of staple retention slots (528ia, 528ib), and wherein the second portion of the second jaw member includes the outer rows of staple retention slots (528oa, 528ob).

5. The loading unit according to claim 4, wherein the staples disposed in the outer rows of staple retention slots (528oa, 528ob) are larger than the staples disposed in the middle row of staple retention slots (528ma, 528mb).

6. The loading unit according to claim 4 or claim 5, wherein the staples disposed in the inner rows of staple retention slots are 4.0 mm staples, the staples disposed in outer rows of staple retention slots are 5.0 mm staples, and the staples disposed in the middle rows of staple retention slots are 4.5 mm staples.

7. The loading unit according to any of claims 4 to 6, wherein the staples disposed in each of the inner, middle and outer rows of staple retention slots in a proximal portion (518a) of the inner side of the curvature of the slot are smaller than the staples disposed in the outer and middle rows of staple retention slots in the remainder of the second jaw member.

8. The loading unit according to claim 7, wherein the staples disposed in each of the inner, middle and outer rows of staple retention slots in the proximal portion of the inner side of the curvature of the slot are 4.0 mm staples.

9. The loading unit according to claim 7 or claim 8, wherein the proximal portion comprises the five most proximal staple retention slots (528₁ₐ - 528₅ₐ) in each of the inner, middle and outer rows of staple retention slots on the inner side of the curvature of the slot.

10. The loading unit according to any of claims 1 to 9, wherein at least some of the staples include a pair of legs (1002a, 1006a) depending from a backspan (1010a), wherein each leg includes a staple tip (1004a, 1008b) defining a first angle α1, and wherein the first angle α1 is between about 25° and about 35°.

11. The loading unit according to any of claims 1 to 10, wherein at least some of the staples include a pair of legs (1002a, 1006a) depending from a backspan (1010a), wherein each leg includes a staple tip (1004c, 1008c) defining a first angle α1 and a second angle α2.

## Patentansprüche

1. Ladeeinheit (500) zur Verwendung mit einem chirurgischen Instrument, wobei die Ladeeinheit umfasst:
einen proximalen Körperteil (502), der konfiguriert ist, um mit einem Abschnitt eines chirurgischen Instruments in Eingriff zu stehen, und eine Längsachse definiert;
ein Paar von Klemmbackenelementen (506, 508), die benachbart zu dem proximalen Körperteil angeordnet sind und sich im Allgemeinen distal davon erstrecken, wobei zumindest eines der Klemmbackenelemente in Bezug auf das andere zwischen einer offenen Position und einer angenäherten Position beweglich ist, zum Eingriff in Körpergewebe dazwischen, wobei das Paar von Klemmbackenelementen ein erstes Klemmbackenelement und ein zweites Klemmbackenelement aufweist; und
eine Vielzahl von Klammern (530), die zumindest teilweise innerhalb des zweiten Klemmbackenelementes angeordnet sind, wobei zumindest einige der Klammern, die in einem ersten Abschnitt des zweiten Klemmbackenelementes angeordnet sind, eine geringere Höhe aufweisen als zumindest einige der Klammern, die in einem zweiten Abschnitt des zweiten Klemmbackenelementes angeordnet sind;
wobei das zweite Klemmbackenelement weiter einen Schlitz (513) umfasst, der konfiguriert ist, um zu ermöglichen, dass sich ein Messer zumindest teilweise dort entlang bewegt, wobei der Schlitz in Bezug auf die Längsachse gebogen ist;
**dadurch gekennzeichnet, dass** der erste Abschnitt des zweiten Klemmbackenelementes (i) auf einer Innenseite der Biegung des Schlitzes und (ii) weiter proximal als der zweite Abschnitt des zweiten Klemmbackenelementes angeordnet ist und der zweite Abschnitt des zweiten Klemmbackenelementes weiter auf einer Außenseite der Biegung des Schlitzes angeordnet ist.

2. Ladeeinheit nach Anspruch 1, wobei jede laterale Seite des Schlitzes des zweiten Klemmbackenelementes eine äußere Reihe von Klammer-Aufbewahrungsschlitzen (528oa, 528ob), eine innere Reihe von Klammer-Aufbewahrungsschlitzen (528ia, 528ib) und eine mittlere Reihe von Klammer-Aufbewahrungsschlitzen (528ma, 528mb) aufweist, wobei die innere Reihe von Klammer-Aufbewahrungsschlitzen dem Schlitz am nächsten liegt, wobei der erste Abschnitt des zweiten Klemmbackenelementes die äußere Reihe von Klammer-Aufbewahrungsschlitzen (528oa), die mittlere Reihe von Klammer-Aufbewahrungsschlitzen (528ma) und die innere Reihe von Klammer-Aufbewahrungsschlitzen (528ia) auf einem Innenabschnitt der Biegung des Schlitzes aufweist, wobei der erste Abschnitt des zweiten Klemmbackenelementes die innere Reihe von Klammer-Aufbewahrungsschlitzen (528ib) auf einem Außenabschnitt der Biegung des Schlitzes aufweist und wobei der zweite Abschnitt des zweiten Klemmbackenelementes die mittlere Reihe von Klammer-Aufbewahrungsschlitzen (528mb) und die äußere Reihe von Klammer-Aufbewahrungsschlitzen (528ob) auf dem Außenabschnitt der Biegung des Schlitzes aufweist.

3. Ladeeinheit nach Anspruch 2, wobei die Klammern, die in der äußeren Reihe der Klammer-Aufbewahrungsschlitze (528ob) auf dem Außenabschnitt der Biegung des Schlitzes angeordnet sind, größer als die Klammern sind, die in der mittleren Reihe von Klammer-Aufbewahrungsschlitzen (528mb) auf dem Außenabschnitt der Biegung des Schlitzes angeordnet sind.

4. Ladeeinheit nach Anspruch 1, wobei jede laterale Seite des Schlitzes des zweiten Klemmbackenelementes eine äußere Reihe von Klammer-Aufbewahrungsschlitzen (528oa, 528ob), eine innere Reihe von Klammer-Aufbewahrungsschlitzen (528ia, 528ib) und eine mittlere Reihe von Klammer-Aufbewahrungsschlitzen (528ma, 528mb) aufweist, wobei die innere Reihe von Klammer-Aufbewahrungsschlitzen dem Schlitz am nächsten liegt; wobei der erste Abschnitt des zweiten Klemmbackenelementes die inneren Reihen von Klammer-Aufbewahrungsschlitzen (528ia, 528ib) aufweist und wobei der zweite Abschnitt des zweiten Klemmbackenelementes die äußeren Reihen von Klammer-Aufbewahrungsschlitzen (528oa, 528ob) aufweist.

5. Ladeeinheit nach Anspruch 4, wobei die Klammern, die in den äußeren Reihen von Klammer-Aufbewahrungsschlitzen (528oa, 528ob) angeordnet sind, größer als die Klammern sind, die in der mittleren Reihe von Klammer-Aufbewahrungsschlitzen (528ma, 528mb) angeordnet sind.

6. Ladeeinheit nach Anspruch 4 oder Anspruch 5, wobei die Klammern, die in den inneren Reihen von Klammer-Aufbewahrungsschlitzen angeordnet sind, 4,0 mm Klammern sind, die Klammern, die in äußeren Reihen von Klammer-Aufbewahrungsschlitzen angeordnet sind, 5,0 mm Klammern sind und die Klammern, die in den mittleren Reihen von Klammer-Aufbewahrungsschlitzen angeordnet sind, 4,5 mm Klammern sind.

7. Ladeeinheit nach einem der Ansprüche 4 bis 6, wobei die Klammern, die in jeder der inneren, mittleren und äußeren Reihen von Klammer-Aufbewahrungsschlitzen in einem proximalen Abschnitt (518a) der Innenseite der Biegung des Schlitzes angeordnet sind, kleiner als die Klammern sind, die in den äußeren und mittleren Reihen von Klammer-Aufbewahrungsschlitzen im Rest des zweiten Klemmbackenelementes angeordnet sind.

8. Ladeeinheit nach Anspruch 7, wobei die Klammern, die in jeder der inneren, mittleren und äußeren Reihen von Klammer-Aufbewahrungsschlitzen in dem proximalen Abschnitt der Innenseite der Biegung des Schlitzes angeordnet sind, 4,0 mm Klammern sind.

9. Ladeeinheit nach Anspruch 7 oder Anspruch 8, wobei der proximale Abschnitt die fünf proximalsten Klammer-Aufbewahrungsschlitze (528₁ₐ - 528₅ₐ) in jeder der inneren, mittleren und äußeren Reihen von Klammer-Aufbewahrungsschlitzen auf der Innenseite der Biegung des Schlitzes umfasst.

10. Ladeeinheit nach einem der Ansprüche 1 bis 9, wobei zumindest einige der Klammern ein Paar Beine (1002a, 1006a) aufweisen, die von einem Rückensteg (1010a) abhängen, wobei jedes Bein eine Klammerspitze (1004a, 1008b) aufweist, die einen ersten Winkel α1 definiert, und wobei der erste Winkel α1 zwischen etwa 25° und etwa 35° liegt.

11. Ladeeinheit nach einem der Ansprüche 1 bis 10, wobei zumindest einige der Klammern ein Paar Beine (1002a, 1006a) aufweisen, die von einem Rückensteg (1010a) abhängen, wobei jedes Bein eine Klammerspitze (1004c, 1008c) aufweist, die einen ersten Winkel α1 und einen zweiten Winkel α2 definiert.

## Revendications

1. Unité de chargement (500) pour une utilisation avec un instrument chirurgical, l'unité de chargement comprenant :
une partie de corps proximale (502) configurée pour mettre en prise une partie d'un instrument chirurgical et définissant un axe longitudinal ;
une paire d'éléments de mâchoire (506, 508) disposés de manière adjacente à la partie de corps proximale et s'étendant de manière généralement distale par rapport à celle-ci, au moins un des éléments de mâchoire étant mobile par rapport à l'autre entre une position ouverte et une position rapprochée pour mettre en prise un tissu corporel entre eux, la paire d'éléments de mâchoire incluant un premier élément de mâchoire et un second élément de mâchoire ; et
une pluralité d'agrafes (530) disposées au moins partiellement à l'intérieur du second élément de mâchoire, dans laquelle au moins certaines des agrafes disposées dans une première partie du second élément de mâchoire ayant une hauteur plus petite qu'au moins certaines des agrafes disposées dans une seconde partie du second élément de mâchoire ;
dans laquelle le second élément de mâchoire comprend en outre une fente (513) configurée pour permettre à un élément coupant de se déplacer au moins partiellement le long de celle-ci, dans laquelle la fente est incurvée par rapport à l'axe longitudinal ;
**caractérisée en ce que** la première partie du second élément de mâchoire est disposée (i) sur un côté interne de la courbure de la fente et (ii) plus éloignée proximalement que la seconde partie du second élément de mâchoire, et la seconde partie du second élément de mâchoire est en outre disposée sur un côté externe de la courbure de la fente.

2. Unité de chargement selon la revendication 1, dans laquelle chaque côté latéral de la fente du second élément de mâchoire inclut une rangée externe de fentes de retenue d'agrafes (528oa, 528ob), une rangée interne de fentes de retenue d'agrafes (528ia, 528ib) et une rangée intermédiaire de fentes de retenue d'agrafes (528ma, 528mb), dans laquelle la rangée interne de fentes de retenue d'agrafes est la plus proche de la fente, dans laquelle la première partie du second élément de mâchoire inclut la rangée externe de fentes de retenue d'agrafes (528oa), la rangée intermédiaire de fentes de retenue d'agrafes (528ma) et la rangée interne de fentes de retenue d'agrafes (528ia) sur une partie intérieure de la courbure de la fente, dans laquelle la première partie du second élément de mâchoire inclut la rangée interne de fentes de retenue d'agrafes (528ib) sur une partie extérieure de la courbure de la fente, et dans laquelle la seconde partie du second élément de mâchoire inclut la rangée intermédiaire de fentes de retenue d'agrafes (528mb) et la rangée externe de fentes de retenue d'agrafes (528ob) sur la partie extérieure de la courbure de la fente.

3. Unité de chargement selon la revendication 2, dans laquelle les agrafes disposées dans la rangée externe de fentes de retenue d'agrafes (528ob) sur la partie extérieure de la courbure de la fente sont plus grandes que les agrafes disposées dans la rangée intermédiaire de fentes de retenue d'agrafes (528mb) sur la partie extérieure de la courbure de la fente.

4. Unité de chargement selon la revendication 1, dans laquelle chaque côté latéral de la fente du second élément de mâchoire inclut une rangée externe de fentes de retenue d'agrafes (528oa, 528ob), une rangée interne de fentes de retenue d'agrafes (528ai, 528ib) et une rangée intermédiaire de fentes de retenue d'agrafes (528ma, 528mb), dans laquelle la rangée interne de fentes de retenue d'agrafes est la plus proche de la fente; dans laquelle la première partie du second élément de mâchoire inclut les rangées internes de fentes de retenue d'agrafes (528ia, 528ib), et dans laquelle la seconde partie du second élément de mâchoire inclut les rangées externes de fentes de retenue d'agrafes (528oa, 528ob).

5. Unité de chargement selon la revendication 4, dans laquelle les agrafes disposées dans les rangées externes de fentes de retenue d'agrafes (528oa, 528ob) sont plus grandes que les agrafes disposées dans la rangée intermédiaire de fentes de retenue d'agrafes (528ma, 528mb).

6. Unité de chargement selon la revendication 4 ou la revendication 5, dans laquelle les agrafes disposées dans les rangées internes de fentes de retenue d'agrafes sont des agrafes de 4,0 mm, les agrafes disposées dans les rangées externes de fentes de retenue d'agrafes sont des agrafes de 5,0 mm et les agrafes disposées dans les rangées intermédiaires de fentes de retenue d'agrafes sont des agrafes de 4,5 mm.

7. Unité de chargement selon l'une quelconque des revendications 4 à 6, dans laquelle les agrafes disposées dans chacune des rangées interne, intermédiaire et externe de fentes de retenue d'agrafes dans une partie proximale (518a) du côté interne de la courbure de la fente sont plus petites que les agrafes disposées dans les rangées externe et intermédiaire de fentes de retenue d'agrafes dans le reste du second élément de mâchoire.

8. Unité de chargement selon la revendication 7, dans laquelle les agrafes disposées dans chacune des rangées interne, intermédiaire et externe de fentes de retenue d'agrafes dans la partie proximale du côté interne de la courbure de la fente sont des agrafes de 4,0 mm.

9. Unité de chargement selon la revendication 7 ou la revendication 8, dans laquelle la partie proximale comprend les cinq fentes de retenue d'agrafes les plus proximales (528₁ₐ-528₅ₐ) dans chacune des rangées interne, intermédiaire et externe de fentes de retenue d'agrafes sur le côté interne de la courbure de la fente.

10. Unité de chargement selon l'une quelconque des revendications 1 à 9, dans laquelle au moins certaines des agrafes incluent une paire de pattes (1002a, 1006a) partant d'un dos (1010a), dans laquelle chaque patte inclut une pointe d'agrafe (1004a, 1008b) définissant un premier angle α1, et dans laquelle le premier angle α1 est entre environ 25° et environ 35°.

11. Unité de chargement selon l'une quelconque des revendications 1 à 10, dans laquelle au moins certaines des agrafes incluent une paire de pattes (1002a, 1006a) partant d'un dos (1010a), dans laquelle chaque patte inclut une pointe d'agrafe (1004c, 1008c) définissant un premier angle α1 et un second angle α2.
